# EUROPÄISCHE PATENTSCHRIFT

(11) **EP 1 968 677 B1**
(45) Veröffentlichungstag und Bekanntmachung des Hinweises auf die Patenterteilung: **08.05.2019**
(21) Anmeldenummer: 06754303.3
(22) Anmeldetag: 12.06.2006
(51) Int. Cl.: A61M 25/00, A61M 5/158, A61M 5/32

(54) **INSERTIONSKOPF MIT NADELSCHUTZ IM GRIFF**
INJECTION HEAD WITH NEEDLE PROTECTION IN THE HANDLE
TÊTE D'INJECTION AVEC UNE PROTECTION D'AIGUILLE DANS LA POIGNÉE

(30) Priorität: 15.09.2005 EP 05020157
(43) Veröffentlichungstag der Anmeldung: 17.09.2008
(73) Patentinhaber: F. Hoffmann-La Roche AG, 4070 Basel (CH); Roche Diabetes Care GmbH, 68305 Mannheim (DE)
(72) Erfinder: SCHEURER, Simon, CH-3006 Bern (CH); THALMANN, Christian, CH-6365 Kehrsiten (CH)
(74) Vertreter: Rentsch Partner AG
(86) Internationale Anmeldenummer: PCT/EP2006/005614
(87) Internationale Veröffentlichungsnummer: WO 2007/031125

(56) Entgegenhaltungen:
- EP-A- 0 615 768
- WO-A-02/081012
- WO-A-2005/046781
- DE-U1-202004 017 862
- FR-A- 2 752 164

## Beschreibung

Die Erfindung betrifft einen Insertionskopf für medizinische oder pharmazeutische Anwendungen, der auf einem organischen Gewebe, vorzugsweise der menschlichen Haut, platzierbar ist und eine Einführeinrichtung aufweist, die in das Gewebe eindringt, wenn der Insertionskopf auf dem Gewebe platziert wird oder gegebenenfalls auch erst nachdem der Insertionskopf auf dem Gewebe platziert wurde. Der Insertionskopf kann insbesondere Bestandteil eines Infusionssets für die Verabreichung eines Medikaments sein.

Aus der DE 198 21 723 ist ein Insertionskopf bekannt mit einer Basis, die eine auf organischem Gewebe platzierbare Unterseite aufweist, über die eine mit der Basis unbeweglich verbundene, flexible Kanüle vorragt, die bei Platzierung der Basis auf der Oberfläche des Gewebes in das Gewebe eindringt. Die Kanüle wird für ein Einstechen in das Gewebe von einer Einstechnadel stabilisiert, welche die Kanüle durchragt. Um einen Benutzer vor Verletzungen durch die vorstehende Einstechnadel und die Einstechnadel mit der Einführeinrichtung vor Beschädigungen zu schützen, ist an der Unterseite des Insertionskopfs ein zylindrischer Nadelschutz lösbar befestigt. Der Nadelschutz benötigt Raum, und es besteht die Gefahr, dass er sich bei unachtsamer Handhabung von dem Insertionskopf löst. Zum Entfernen des Nadelschutzes benötigt der Benutzer ferner beide Hände, wobei sich die eine Hand nach Entfernen des Nadelschutzes noch in unmittelbarer Nähe der dann frei vorstehenden Einstechnadel befindet.

Aus der deutschen Patentanmeldung Nr. 10 2004 039 408.3 ist ein Insertionskopf mit einer Einführeinrichtung bekannt, die aus einer Schutzposition, in der sie in einer Aufnahme aufgenommen ist, in eine Einführposition bewegbar ist, in der sie über die Unterseite des Insertionskopfs vorragt und bei dem Aufsetzen des Insertionskopfs auf der Gewebeoberfläche in das Gewebe eindringt. Die Aufnahme ist integraler Bestandteil des Insertionskopfs, der hierfür einen zur Unterseite offenen Hohlraum bildet. In der Schutzposition ist die Einführeinrichtung vollständig in dem die Aufnahme bildenden Hohlraum aufgenommen, insbesondere ragt ihr freies Ende nicht über die Unterseite oder seitlich vor. Der Insertionskopf benötigt vorteilhafterweise zwar keinen gesonderten Schutz für die Einführeinrichtung und ist deshalb im Auslieferungszustand so flach wie im Gebrauchszustand, d.h. wie in dem auf dem Gewebe platzierten Zustand. Wegen der integriert gebildeten Aufnahme verlängert sich jedoch der Insertionskopf.

Die DE 20 2004 017 862 U1 bezieht sich auf eine Auszieheinrichtung zum geschützten Herausziehen einer flexiblen Punktionsnadel aus einem Katheter, insbesondere einem Katheter mit flexiblem Verweilschlauch, nach dem Punktieren, mit einem Verbindungsabschnitt zum Verbinden der Auszieheinrichtung mit dem Katheter in einem Gebrauchsanfangszustand, einer Gleitschiene und einem in der Gleitschiene linear bewegbaren Gleiter, wobei der Gleiter einen Mitnehmerabschnitt aufweist und wobei die Punktionsnadel mit einem aus dem Katheter hervorragenden Ende an der Gleitschiene derart festgelegt/festlegbar ist, dass sie mit einer Schlaufe um den Mitnehmerabschnitt des Gleiters herumgeführt ist und an diesem anliegt.

Die WO 02/081012 A2 bezieht sich auf eine Vorrichtung zum gemeinsamen Einbringen einer Kanüle und einer Punktionsnadel in Gewebe. Ein Schutzelement nimmt die Kanüle und die Punktionsnadel in einem Ausgangszustand auf. Mittels eines Betätigungselement werden Kanüle und Punktionsnadel aus dem Schutzelement herausbewegt. Anschliessend wird die Punktionsnadel zurückgezogen, während die Kanüle im Gewebe verbleibt.

Die FR 2 752 164 bezieht sich auf eine Nadel, um Haut zu durchstechen. Die Nadel ist mit einem Zufuhrrohr verbunden und drehbar in einem Gehäuse gelagert, und zwar zwischen einer Position, um die Haut zu durchstechen und einer eingezogenen Position. Ein Betätigungselement in Form eines Druckknopfes bewegt die Nadel mittels einer sich im Gehäuse drehenden Welle. Die Welle hat zwei Nocken,
und eine Nocke wird durch den Knopf bewegt, um die Welle zu drehen und die Nadel aus dem Gehäuse zu bewegen. Die andere Nocke wird bewegt, wenn der Knopf freigegeben ist und die dient zum Einziehen der Nadel.

Es ist eine Aufgabe der vorliegenden Erfindung, einen Insertionskopf zu schaffen, der gefahrlos handhabbar ist, und im Gebrauchszustand, d.h. nach Platzierung auf einer Gewebeoberfläche und eingedrungener Einführeinrichtung, kompakt ist.

Diese Aufgabe wird erfindungsgemäß durch den Gegenstand des Patentanspruchs 1 gelöst. Die Unteransprüche beschreiben bevorzugte Ausführungsformen.

Ein Insertionskopf, wie die Erfindung ihn betrifft, umfasst eine Basis mit einer auf organischem Gewebe platzierbaren Unterseite, eine in das Gewebe einführbare Einführeinrichtung und eine Aufnahme, die zumindest ein freies Ende der Einführeinrichtung in einer Schutzposition der Einführeinrichtung aufnimmt.

Nach der Erfindung ragt in der Schutzposition der Einführeinrichtung zumindest deren freies Ende nicht über die Unterseite der Basis vor. Vielmehr wird die Einführeinrichtung von der Basis beweglich gelagert, so dass sie relativ zu der Basis aus der Schutzposition in eine Einführposition bewegbar ist, in der ihr freies Ende über die Unterseite vorsteht.

Vorzugsweise steht die Einführeinrichtung in der Schutzposition über ihre gesamte Länge hinter der Unterseite der Basis zurück und wird vollständig abgeschirmt, vorzugsweise auch blickdicht verdeckt. In bevorzugten Ausführungen weist die Einführeinrichtung in der Schutzposition zumindest im Wesentlichen parallel zu der Unterseite bzw. der Auflagefläche der Basis. Dies begünstigt eine flache Bauweise der Basis, wobei deren Höhe rechtwinklig zur Unterseite gemessen wird. Ferner ist die Aufnahme mit der Basis lösbar verbunden. Im verbundenen Zustand schafft die Aufnahme den Schutz für die Einführeinrichtung, so dass die Basis von dieser Funktion entlastet ist und daher bei der Basis die sonst zur Erfüllung der Schutzfunktion erforderliche Länge eingespart wird. Die Aufnahme ist zu der Unterseite der Basis hin offen. Grundsätzlich würde eine Öffnung nur für die Einführeinrichtung genügen, vorzugsweise umgibt die lösbare Aufnahme jedoch die Basis oder einen Teil der Basis topf- oder schüsselartig, vorzugsweise der Form nach wie ein halber Topf oder eine halbe Schüssel. Die Aufnahme kann nach Lösen einer Verbindung vorzugsweise einfach nach oben von der Basis abgenommen werden.

Zur Erleichterung der Handhabung ist der Insertionskopf mit einem von der Basis abragenden Griff ausgestattet. Der Griff oder im Falle eines bevorzugt mehrteiligen Griffes zumindest eine Griffkomponente ist mit der Basis lösbar verbunden. Dabei bildet ein solcher Griff oder eine solche Griffkomponente die lösbare Aufnahme für die Einführeinrichtung. In bevorzugter Ausführung weist der Griff einen von der Oberseite der Basis aufragenden Griffteil auf, von dem die Aufnahme seitlich abragt.

Vorzugsweise ist der Griff mehrteilig und umfasst eine Griffkomponente, die relativ zu der Basis und einer weiteren Griffkomponente oder gegebenenfalls mehreren weiteren Griffkomponenten beweglich ist. Die bewegliche Griffkomponente ist mit der Einführeinrichtung vorzugsweise so gekoppelt, dass eine Bewegung dieser Griffkomponente die Bewegung der Einführeinrichtung bewirkt. Eine weitere Griffkomponente, die vorzugsweise ein Widerlager für das Bewegen der beweglichen Griffkomponente bildet, kann relativ zu der Basis im verbundenen Zustand unbeweglich sein und vorteilhafterweise die lösbare Aufnahme bilden. Grundsätzlich kann stattdessen die bewegliche Griffkomponente die lösbare Aufnahme bilden. Mehrere Griffkomponenten können die Aufnahme auch erst gemeinsam bilden.

Aufgrund der Ausstattung des Griffs mit einer beweglichen Griffkomponente kann die Bewegung der Einführeinrichtung allein durch Greifen und Betätigen des Griffs bewirkt werden. Der Griff bildet selbst das Widerlager für die bewegbare Griffkomponente. In diesem Sinne wird hier der das Widerlager bildende Teil des Griffs als weitere Griffkomponente bezeichnet. Die bewegliche Griffkomponente kann beispielsweise als Druckknopf gebildet sein. Die weitere Griffkomponente kann ein Gehäuse sein, aus dem solch ein Druckknopf herausragt. In ebenfalls bevorzugten Ausführungen bilden die beiden

Griffkomponenten erst gemeinsam den Griff, beispielsweise die Hälften eines insgesamt zweiteiligen Griffs.

Für die automatisierte Platzierung mittels des erfindungsgemäßen Inserters weist der Insertionskopf, vorzugsweise dessen Griff, eine Haltestruktur auf, die mit der Halteeinrichtung des Inserters in dem Halteeingriff ist. Falls der Griff wie bevorzugt eine mit der Basis unbeweglich verbundene Griffkomponente und eine relativ zu dieser Griffkomponente bewegliche Griffkomponente aufweist, bildet vorzugsweise die unbewegliche Griffkomponente die Haltestruktur. Die Haltestruktur kann an dem Griff oder der unbeweglichen Griffkomponente insbesondere in einem Stück angeformt sein, vorzugsweise ist sie mit dem Griff oder der unbeweglichen Griffkomponente zumindest steif verbunden.

Die bewegliche Griffkomponente bildet vorzugsweise das vorstehend in Bezug auf das System aus Insertionskopf und Inserter genannte Empfangsglied, wird jedoch nachfolgend als bewegliche Griffkomponente bezeichnet, solange besonders bevorzugte Ausführungen des Insertionskopfs beschrieben werden. Grundsätzlich muss das Empfangsglied des Systems jedoch keine Griffkomponente sein, sondern kann ausschließlich der Kopplung zwischen dem Aktivierungsglied und der Einführeinrichtung dienen.

In bevorzugten Ausführungen ist die bewegliche Griffkomponente zumindest im Wesentlichen parallel zu der Unterseite der Basis bewegbar. Insbesondere kann es sich um eine Linearbeweglichkeit handeln. Alternativ zu einer reinen Translationsbeweglichkeit kann die zweite Komponente beispielsweise schwenkbeweglich angebracht sein.

Die wenigstens eine weitere Griffkomponente ist mit der Basis vorzugsweise nicht beweglich verbunden. Grundsätzlich wäre es jedoch auch denkbar, dass beide Griffkomponenten relativ zu der Basis beweglich mit dieser verbunden sind.

Für die Übertragung der Bewegung der beweglichen Griffkomponente auf die Einführeinrichtung kann eine steife Kopplung vorgesehen sein, d.h. die bewegliche Griffkomponente und die Einführeinrichtung können steif miteinander verbunden sein, worunter auch eine Urformung in einem Stück verstanden wird. Eine steife Kopplung kann beispielsweise im Falle einer bevorzugten Schwenkbeweglichkeit der Einführeinrichtung ohne weiteres dann verwirklicht werden, wenn auch die bewegliche Griffkomponente schwenkbeweglich ist. Gegenüber dem Insertionskopf der deutschen Patentanmeldung Nr. 10 2004 039 408.3 weist der erfindungsgemäße Insertionskopf dennoch den Vorteil auf, dass die weitere Griffkomponente dem Benutzer als Widerlager dienen kann und nicht das Gewebe über die Basis die für die Schwenkbewegung aufzubringende Kraft aufnehmen muss, vielmehr wird diese Kraft durch das Halten der weiteren Griffkomponente vom Benutzer aufgenommen. Verfügt der Benutzer über einen Inserter, insbesondere den erfindungsgemäßen, mit dem der Insertionskopf auf dem Gewebe platziert und dabei die Einführrichtung eingeführt wird, nimmt der Inserter diese Kraft auf.

In bevorzugten Ausführungen sind die bewegliche Griffkomponente und die Einführeinrichtung über ein Getriebe miteinander gekoppelt. Eine derartige Kopplung hat den Vorteil, dass die Beweglichkeit der Griffkomponente nicht der Beweglichkeit der Einführeinrichtung entsprechen muss, sondern beide Beweglichkeiten jeweils einzeln für sich optimal gestaltet werden können. So kann die Einführeinrichtung insbesondere schwenkbeweglich und die bewegliche Griffkomponente translatorisch beweglich und dabei vorzugsweise linear geführt sein. Im Falle einer Schwenkbeweglichkeit auch der Griffkomponente kann deren Schwenkachse eine andere als die der Einführeinrichtung sein. Während die Einführeinrichtung vorzugsweise um eine zu der Unterseite der Basis zumindest im wesentlichen parallelen Rotationsachse schwenkbar ist, was im übrigen für sämtliche Ausführungsformen der beweglichen Griffkomponente gilt, kann eine schwenkbewegliche Griffkomponente um eine zur Unterseite zumindest im wesentlichen rechtwinkligen Rotationsachse schwenkbeweglich sein. Ein Getriebe kann aber auch dann von Vorteil sein, wenn im Falle einer schwenkbeweglichen Griffkomponente deren Rotationsachse von der Rotationsachse der schwenkbeweglichen Einführeinrichtung parallel beabstandet ist. In solch einem Fall kann der Schwenkwinkel der Griffkomponente mittels eines Getriebes untersetzt oder vorzugsweise übersetzt auf die Einführeinrichtung übertragen werden.

Eine bevorzugte getriebetechnische Kopplung umfasst ein Zahnrad und eine Zahnstange, die miteinander in einem Zahneingriff sind und bei Bewegung der Griffkomponente miteinander kämmen. Die Zahnstange ist vorzugsweise mit der beweglichen Griffkomponente verbunden, so dass eine Bewegung der Griffkomponente in Längsrichtung der Zahnstange in eine Drehbewegung des in diesem Fall mit der Einführeinrichtung verbundenen Zahnrads übertragen wird. Bei entsprechend feiner Verzahnung bzw. Zahnteilung kann ein vergleichsweise kurzer Hub der beweglichen Griffkomponente in eine Drehbewegung des Zahnrads über einen beträchtlichen Teil einer vollen Umdrehung, vorzugsweise in eine viertel Umdrehung des Zahnrads, übertragen werden. Vorteilhafterweise ist die bewegliche Griffkomponente mit der Zahnstange in einem Stück geformt. Anstatt einer Kopplung über ein oder gegebenenfalls mehrere Zahnpaarungen, kann die Kopplung auch als Führungskurve und Eingriffsglied gebildet sein oder ein solches Führungskurvengelenk umfassen, obgleich eine Kopplung mittels Zahneingriffs der Vorzug gegeben wird.

Der Griff ist in bevorzugten Ausführungen wie bereits erwähnt lösbar mit der Basis verbunden. Bevorzugt löst sich diese Verbindung bei der Bewegung der Einführeinrichtung in die Einführposition automatisch, besonders bevorzugt bei der Bewegung der beweglichen Griffkomponente. Alternativ wäre es grundsätzlich jedoch ebenfalls denkbar, den Griff oder die Basis mit einer weiteren beweglichen Komponente auszustatten, durch deren Betätigung die Verbindung mit der Basis gelöst wird. Die Verbindung zwischen dem Griff und der Basis kann zwar durch einen reinen Reibschluss hergestellt sein, bevorzugter beruht sie jedoch ausschließlich auf einem Formschluss oder einer Kombination aus Form- und Reibschluss. Um die Verbindung zu schaffen, sind die Basis und der Griff je mit wenigstens einem Verbindungselement ausgestattet, die bei bestehender Verbindung miteinander in einem Eingriff sind. Um die Verbindung lösen zu können, ist vorzugsweise wenigstens eines der Verbindungselemente gegen eine rückstellende Elastizitätskraft aus dem Eingriff bewegbar. In bevorzugten Ausführungen dient die bewegliche Griffkomponente nicht nur der Überführung der Einführeinrichtung in die Einführposition, sondern auch dem Lösen der Verbindung, indem die zweite Griffkomponente bei ihrer Bewegung durch Kontakt, vorzugsweise Gleitkontakt, eines der Verbindungselemente gegen die Elastizitätskraft aus dem Eingriff bewegt, beispielsweise elastisch abbiegt. Obgleich weniger bevorzugt, kann die weitere Griffkomponente jedoch alternativ in einem Stück mit der Basis geformt oder unlösbar an der Basis befestigt sein. In solch einer Ausführung sollte sie jedoch möglichst kurz sein.

Falls der Insertionskopf nicht über einen besonderen, d.h. ausgeprägten Griff verfügt oder die lösbare Aufnahme nicht Bestandteil eines derartigen Griffs ist, beispielsweise indem sie an dem Griff in einem Stück angeformt ist, wird es dennoch bevorzugt, wenn durch die für das Lösen der Aufnahme erforderlichen Handgriffe automatisch auch die Einführeinrichtung aus der Schutzposition in die Einführposition bewegt wird. So kann an der lösbaren Aufnahme beispielsweise ein Druckknopf oder ein anderes Betätigungselement angebracht oder ein solches Element mit der Aufnahme geeignet verbunden sein und die Aufnahme durch Betätigung des Betätigungselements von der Basis gelöst werden.

Die Einführeinrichtung kann eine biegesteife Kanüle oder Nadel sein. Bevorzugt ist die Einführeinrichtung zumindest in dem Gewebe flexibel. Insbesondere kann die Einführeinrichtung eine Biegesteifigkeit aufweisen, die sich im eingeführten Zustand aufgrund einer zwischen dem Material der Einführeinrichtung und dem umgebenden Gewebe stattfindenden Wechselwirkung verringert. Die Einführeinrichtung kann alternativ auch in herkömmlicher Weise flexibel gebildet sein, beispielsweise als flexible Kanüle, und von einer biegesteifen Einstecheinrichtung während der Einführung in das Gewebe stabilisiert werden. Die Einführeinrichtung ist vorzugsweise in eine Einführrichtung langgestreckt und vorzugsweise schlank.

Falls die Einführeinrichtung von Hause aus flexibel ist, d.h. nicht erst durch Wechselwirkung mit dem Gewebe flexibel wird, wird sie vorzugsweise mittels einer Einstecheinrichtung stabilisiert, um zu verhindern, dass die Einführeinrichtung bei dem Einführen in das Gewebe knickt. Die Einstecheinrichtung kann insbesondere als dünne Einstechnadel gebildet sein. Wenn die Einführeinrichtung in das Gewebe eingeführt worden ist, wird die Einstecheinrichtung vorteilhafterweise entfernt. Das Entfernen einer derartigen Einstecheinrichtung wird vorzugsweise durch Entfernen der lösbaren Aufnahme bewirkt, beispielsweise mit dem Griff bewerkstelligt. Falls die Einstecheinrichtung wie bevorzugt in der Schutzposition noch nicht mit der lösbaren Aufnahme verbunden ist, verbindet sie sich bei der Bewegung in die Einführposition vorzugsweise automatisch mit der Aufnahme oder einem damit verbundenen Teil. Hierfür kann an ihrem von dem freien Ende der Einführeinrichtung abgewandten Ende ein Verbindungselement, vorgesehen sein, das zugleich mit dem Abschluss der Bewegung in die Einführposition oder kurz zuvor mit einem Verbindungsgegenelement, das mit der lösbaren Aufnahme verbunden ist in einen Verbindungseingriff gelangt. Die Verbindung kann zwar grundsätzlich rein reibschlüssig sein, vorzugsweise umfasst sie jedoch einen Formschluss zumindest. Das Verbindungselement der Einstecheinrichtung kann mit dem Verbindungsgegenelement insbesondere eine Schnappverbindung bilden. Grundsätzlich genügt für eine formschlüssige Verbindung auch nur ein einfacher Hintergriff bezüglich der Richtung, in welche die lösbare Aufnahme von der Basis entfernt werden soll; ein elastischer Schnappeingriff ist daher nicht unumgänglich erforderlich.

Die Einführeinrichtung ragt in der Einführungsposition vorzugsweise von der Unterseite des Gehäuses vor. Grundsätzlich kann sie jedoch stattdessen auch von einer Seite des Gehäuses vorragen, solange sie für ein Eindringen in das Gewebe über die Unterseite ausreichend weit vorragt. Die Einführeinrichtung ragt in der Einführposition vorzugsweise mit einer an subkutane Applikationen angepassten Länge über die Unterseite des Gehäuses vor, vorzugsweise unmittelbar von der Unterseite ab oder aus der Unterseite hervor. Für Applikationen innerhalb der Haut oder in intramuskulärem Gewebe ist die Einführeinrichtung entsprechend kürzer oder länger. Als Einführeinrichtung wird derjenige Längenabschnitt verstanden, der in der Applikation in das Gewebe ragt.

In bevorzugten Ausführungen ist die Einführeinrichtung mit der Basis bewegbar permanent verbunden. Dass die Einführeinrichtung mit der Basis permanent verbunden ist, bedeutet, dass sie zumindest für die Bewegung aus der Schutzposition in die Einführposition und/oder aus der Einführposition in die Schutzposition nicht von der Basis gelöst wird. Vorzugsweise ist die Einführeinrichtung sogar in dem Sinne permanent mit der Basis verbunden, dass sie von der Basis gar nicht gelöst werden kann, zumindest nicht ohne eine besondere Kraftanstrengung oder nur durch Zerstörung.

Erfindungsgemäss ist die Einführeinrichtung mit der Basis so verbunden, dass die Einführeinrichtung relativ zu der Basis schwenkbar ist. Vorteilhafterweise, jedoch nur optional, kann die Einführeinrichtung oder zumindest deren freies Ende nach einem Ausschwenken wieder in die lösbare Aufnahme eingeschwenkt werden. In derartigen Ausbildungen bilden die Basis und ein mit der Einführeinrichtung verbundenes Gelenkelement ein Drehgelenk. Die Einführeinrichtung ragt von dem Gelenkelement vorzugsweise einfach quer zu der Rotationsachse des Gelenks ab, beispielsweise indem sie an das Gelenkelement angeformt ist, oder sie kann grundsätzlich auch erst wieder gelenkig mit dem Gelenk verbunden sein. In der Lage, welche die Einführeinrichtung gerade einnimmt, wenn ihr freies Ende über die Unterseite vorschwenkt, schliesst die Längsachse der Einführeinrichtung mit der Unterseite einen spitzen Winkel von vorzugsweise weniger als 50° ein. Vorzugsweise ist der Winkel kleiner als 30°, so dass die Längsachse bzw. die Einführeinrichtung im Moment des Ausschwenkens zumindest im Wesentlichen parallel zu der Unterseite bzw. der Auflageflache der Basis weist. Die Längsachse der Einführeinrichtung, die um die Rotationsachse geschwenkt wird, schneidet die Rotationsachse vorzugsweise. Falls die Längsachse der Einführeinrichtung die Rotationsachse nicht schneidet, sondern in einem Abstand kreuzt, ist der Abstand vorzugsweise deutlich kleiner als die Länge der Einführeinrichtung. Vorzugsweise ist der Abstand höchstens halb so gross wie die Eindringtiefe bzw. Länge der Einführeinrichtung. Der Schwenkwinkel der Einführeinrichtung beträgt in bevorzugten Ausführungen 90° ± 10°. In ebenfalls vorteilhaften Ausführungen kann der Schwenkwinkel jedoch auch kleiner sein, insbesondere falls die Einführeinrichtung in der Einführposition nicht rechtwinklig zur Unterseite der Basis weist, sondern in einem spitzen Winkel, der allerdings wenigstens 30° betragen sollte. Entsprechend beträgt der Schwenkwinkel in derartigen Ausführungen vorzugsweise wenigstens etwa 30° oder jeden Zwischenwert zwischen etwa 30° und etwa 90°. Grundsätzlich kann der Schwenkwinkel auch grösser als 90° sein.

In einer alternativen, nicht anspruchsgemässen Ausführungsform kann die Basis für die Einführeinrichtung eine Verschiebeführung bilden, an der die Einführeinrichtung bei ihrer Bewegung aus der Schutzposition in die Einführposition und/oder aus der Einführposition in die Schutzposition translatorisch geführt wird. In einer derartigen Ausführung bilden die Basis und ein Gelenkelement, das mit der Einführeinrichtung verbunden ist, ein Schubgelenk.

Auch von einem derartigen Gelenkelement ragt die Einführeinrichtung in bevorzugter Ausführung einfach ab, vorzugsweise in Schubrichtung. In noch einer Alternative weist die Einführeinrichtung oder eine sie stabilisierende Einstecheinrichtung eine derartige Biegesteifigkeit auf, die es ermöglicht, die Einführeinrichtung oder gegebenenfalls eine sie stabilisierende Einstecheinrichtung mit dem jeweils freien Ende zu der Unterseite der Basis hin in die Aufnahme zu biegen, so dass das freie Ende in die Aufnahme hineinragt und in der Aufnahme mit der Basis lösbar verrastet oder anderweitig in diesem Biegezustand gegen ihre rückstellende Elastizität lösbar gehalten wird. Eine derartige Ausführung ist besonders dann denkbar, wenn die Einführeinrichtung in der Einführposition mit der Unterseite der Basis einen spitzen Winkel einschließt. Denkbar ist auch, dass für ein derartiges Einbiegen die Einführeinrichtung ein Stück weit hinter der Unterseite der Basis an der Basis oder der lösbaren Aufnahme befestigt ist, um bereits innerhalb eines Hohlraums des Insertionskopfs eine gewisse Biegelinie zu erhalten. Andererseits ist es vorteilhaft, wenn die Einführeinrichtung in der Einführposition unmittelbar oder möglichst nahe von der die Gewebeoberfläche im Gebrauch kontaktierenden Unterseite vorragt, d.h. an der Unterseite oder in einem möglichst kurzen Abstand von der Unterseite so eingespannt ist, vorzugsweise fest oder gegebenenfalls axial beweglich und ansonsten fest, das eine beim Eindringen in das Gewebe biegbare Länge der Einführeinrichtung oder einer sie stabilisierenden Einstecheinrichtung möglichst kurz ist. Eine in der Einführposition unmittelbar von oder nahe der Unterseite abragende Einführeinrichtung wird jedenfalls dann bevorzugt, wenn die Einführeinrichtung für die Schutzposition nicht verbogen wird.

Obgleich es genügt, wenn die Einführeinrichtung nur in eine Richtung, d.h. einmalig, relativ zu der Basis bewegbar ist, dann vorzugsweise aus der Schutzposition in die Einführposition, kann es vorteilhaft sein, wenn die Einführeinrichtung zwischen den beiden Positionen hin und her bewegbar ist, so dass die Einführeinrichtung für einen Gebrauch in die Einführposition und für eine Entsorgung wieder in die Schutzposition bewegt werden kann. Die Verbindung zwischen der Einführeinrichtung und dem Gehäuse ist oder umfasst vorzugsweise einen Formschluss, der insbesondere ein Gelenk bilden kann. Die Verbindung kann alternativ auch rein kraftschlüssig oder sogar rein stoffschlüssig sein, beispielsweise dann, wenn die Einführeinrichtung in der Schutzposition gebogen ist.

Der Insertionskopf ist vorzugsweise Bestandteil eines Infusionssets für die Verabreichung von Insulin, eines Schmerzmittels oder eines anderen per Infusion verabreichbaren Medikaments oder ist für eine derartige Verwendung vorgesehen. Anstatt für eine Medikamentenverabreichung oder grundsätzlich auch eines anderen verabreichbaren Produkts kann der Insertionskopf auch zu Diagnosezwecken dienen. In solchen Applikationen kann die Einführeinrichtung Träger eines Sensors zum Messen von beispielsweise der Glukosekonzentration in einer Körperflüssigkeit oder einer anderen physikalischen und/oder biochemischen Größe dienen, die für den Gesundheitszustand eines Patienten maßgeblich ist oder sein kann. Der Insertionskopf kann zu Diagnosezwecken auch als Perfusionsvorrichtung gebildet sein. In solch einer Ausbildung wird die Einführeinrichtung nach dem Einführen in das Gewebe von einer Spülflüssigkeit durchströmt, die ein oder mehrere bestimmte Inhaltsstoffe der Körperflüssigkeit bei dem Durchströmen aufnimmt, um die mit dem betreffenden Inhaltsstoff oder die mehreren Inhaltsstoffen angereicherte Spülflüssigkeit zu analysieren. Schließlich kann der Insertionskopf eine Vorrichtung für die Verabreichung eines Produkts und eine Diagnoseeinrichtung in Kombination bilden. Die Einführeinrichtung kann für die Zuführung eines Produkts, dass insbesondere ein Medikament oder eine Spülflüssigkeit sein kann, oder die Abführung einer Körperflüssigkeit oder nur eines oder mehrerer Inhaltsstoffe einer Körperflüssigkeit angepasst geformt sein. In derartigen Applikationen bildet die Einführeinrichtung wenigstens einen Strömungsquerschnitt. Die Einführeinrichtung kann der Zu- und Abführung von Stoffen auch in Kombination dienen. Ist der Insertionskopf nur als Messvorrichtung gebildet, so kann die Einführeinrichtung auch nur dazu dienen, einen Sensor oder einen Teil eines Sensors zu platzieren, d.h. rein als mechanische Einführeinrichtung. In einer Weiterbildung als Messvorrichtung kann der Insertionskopf über das mechanische Eindringen hinaus auch der Übertragung von Steuersignalen zu dem Sensor und/oder von Messsignalen von dem Sensor dienen. In kombinierten Applikationen kann er schließlich über wenigstens einen Strömungsquerschnitt für den Stofftransport, d.h. eine Strömungsleitung, und wenigstens eine Signalleitung verfügen. Auf die Signalleitung kann verzichtet werden, wenn der Sensor für den drahtlosen Empfang von Steuersignalen und/oder das drahtlose Senden von Messsignalen eingerichtet ist. Schließlich kann die Einführeinrichtung auch zwei oder mehr Einführelemente ausweisen, die separat abragen. So kann ein erstes Einführelement den Stofftransport in das Gewebe und ein anderes, zweites Einführelement dem Stofftransport aus dem Gewebe oder nur dem Eindringen eines Sensors oder eines Teils eines Sensors dienen. Mit mehreren Einführabschnitten, die je einen Strömungsabschnitt aufweisen, können mit dem gleichen Insertionskopf auch unterschiedliche Stoffe verabreicht werden. Dies kann auch mit einer Einführeinrichtung verwirklicht werden, die mehrere, separate Strömungsquerschnitte in einem gemeinsamen Abschnitt bildet.

Gemäß einer weiteren bevorzugten Ausführungsform ist es möglich, den erfindungsgemäßen Insertionskopf in der Aufnahme, die die Einführeinrichtung nebst Einstechnadel oder diese allein in der Schutzposition oder ggf. einer anderen Position, wie etwa einer Sperrposition, aufnimmt, eine Sicherungskulisse vorzusehen, in die die Einführeinrichtung ggf. in Verbindung mit der Einstechnadel oder die Einstechnadel allein beispielsweise in der Schutzposition in der Aufnahme einführbar ist. Durch die federnde Eigenschaft der Einstechnadel kann die Spitze der Einstechnadel entlang der Sicherungskulisse beim Zurückschwenken nach dem Gebrauch in die Schutzposition bzw. in die Aufnahme elastisch verformt bzw. seitlich ausgelenkt werden, um anschließend in eine Sicherungsausnehmung hinter einer Sicherungsschulter einzurasten. In dieser Position wird dann die Einstechnadel sicher in der Schutzposition gehalten. Eine Verletzung durch die kontaminierte Einstechnadel kann dann nicht mehr auftreten.

Gemäß einer weiteren bevorzugten Ausführungsform kann die bewegliche Griffkomponente in die weitere Griffkomponente so weit einschiebbar sein, dass eine Steuerrampe an der beweglichen Griffkomponente nach Beendigung des Vorgangs zur Überführung der Einführeinrichtung von der Schutzposition in die Einführposition gegen eine Auslenkrampe an einem Verbindungselement, das die Basis reversibel mit dem Griff verbindet, anlaufen, um das Eingriffselement aus seiner Eingriffsstellung auszulenken, so dass der Griff und die Basis trennbar sind. Diese Ausbildung insbesondere in Verbindung mit der zweiten Strecke der Kulissenführung ist besonders vorteilhaft, um ohne Beanspruchung des Gewebes des Patienten den erfindungsgemäßen Insertionskopf von der Einstechfunktion in die Zuführfunktion zum Zuführen eines Medikaments zu überführen.

Gemäß einer weiteren vorteilhaften Ausführungsform kann der Einführeinrichtung eine Haltestruktur zugeordnet sein, die mit der Einführeinrichtung mitbewegt wird, wobei die Haltesstruktur eine Rastschulter aufweist, die beim Herausziehen der weiteren Griffkomponente aus der beweglichen Griffkomponente in einen verrastenden Eingriff mit einem Eingriffelement tritt, um ein neuerliches Herausschwenken der Einstecheinrichtung verhindern zu können.

Gemäß einer bevorzugten Ausführungsform ist die Aufnahme einstückig mit dem Griff oder der weiteren Griffkomponente ausgebildet. In diesem Fall ist die Aufnahme getrennt von der Basis vorgesehen.

Ferner kann in vorteilhafter Weise der Einführeinrichtung eine Haltestruktur zugeordnet sein, die mit der Einführeinrichtung mitbewegt wird, wobei die Haltestruktur eine Sicherungsstruktur umfasst. Diese sollte einer parallel zu der Bewegungsrichtung der beweglichen Griffkomponente erstreckten Wand der beweglichen Griffkomponente gegenüberliegen. Dadurch wird es einem Eingriffselement an der Innenseite der Wand mit der Sicherungsstruktur ermöglicht, in der Schutzposition für die Einführeinrichtung im reversiblen Eingriff zu stehen. Mittels dieser Maßnahmen ist es möglich, die Einführeinrichtung in Verbindung mit der Einstechnadel sicher in der Schutzposition in der Aufnahme zu halten, um anschließend mit der beweglichen Griffkomponente dennoch nach Überwindung eines anfänglichen Widerstandes, der durch die Sicherungsstruktur und das Eingriffselement bereitgestellt wird, die gewünschte Schwenkbetätigung für die Einführeinrichtung in Verbindung mit der Einstechnadel bewerkstelligen zu können.

Gemäß einer weiteren bevorzugten Ausführungsform der Erfindung kann ein Insertionskopf mit einer Einführeinrichtung versehen sein, die über eine Dreh- oder Schwenkachse an der Basis gehalten ist. Ein Kulissenstein oder Zapfen kann vorgesehen sein, der in Bezug auf die Drehachse azentrisch und bevorzugt getrennt von dieser angeordnet ist, wobei eine Kulissenführung vorgesehen ist, die im Wirkzusammenhang mit dem Kulissenstein steht. Eine Bewegung der beweglichen Griffkomponente relativ zu der Basis über eine erste Strecke eines ersten Abschnitts der Kulissenführung überführt den Kulissenstein aus einer ersten Stellung, die zu der Schutzposition der Einführeinrichtung bzw. Nadel entspricht, in eine zweite Stellung, die der Einführposition der Einführeinrichtung korrespondiert. Diese Ausbildung ermöglicht eine relativ komplexe Führungsstrecke für die Einführeinrichtung im Herausschwenken aus der Aufnahme, wobei gleichzeitig relativ geringe Kräfte über die bewegliche Griffkomponente ausgeübt werden müssen und ein Verkanten zwischen den einzelnen Bestandteilen der Mechanik höchst unwahrscheinlich ist.

Gemäß einer anderen vorteilhaften Ausführungsform der Erfindung kann ein Verbindungselement vorgesehen sein, das die Basis reversibel mit dem Griff verbindet, wobei zum Lösen des Griffs von der Basis der Griff über eine zweite Strecke senkrecht zur Erstreckungsrichtung der in der Einführposition befindlichen Einführeinrichtung bewegbar ist, wobei die Kulissenführung einen zu der zweiten Strecke korrespondierenden zweiten Abschnitt aufweist, so dass die Einführeinrichtung beim Lösen nicht mitbewegt wird. D. h., durch die passende Ausbildung der Kulissenführung ist es möglich, nach dem Einstechen der Einstechnadel und der Einführeinrichtung in den Körper eines Patienten eine Bewegung des Griffes parallel zur Körperoberfläche bzw. zur Oberfläche der Lasche, nachfolgend auch als Pflaster bezeichnet, zu bewerkstelligen, ohne dass dabei das organische Gewebe durch parallele Kräfte strapaziert wird. Auf diese Weise ist eine Trennung des Griffbereichs von der Basis und damit der Einstechnadel von der Einführeinrichtung in vorteilhafter Weise durch Betätigen derselben Komponente möglich, und hier insbesondere der beweglichen Griffkomponente, um sowohl die Auslösung als auch die Entkopplung vorzunehmen.

Damit ist es möglich, gemäß der Erfindung die kontaminierte Einstecheinrichtung bzw. Einstechnadel wieder in die bewegliche Griffkomponente zurückzuschwenken.

Gemäß einer weiteren bevorzugten Ausführungsform der Erfindung kann ein Insertionskopf mit einer Einführeinrichtung versehen sein, die über eine Dreh- oder Schwenkachse an der Basis gehalten ist. Ein Kulissenstein kann vorgesehen sein, der in Bezug auf die Drehachse azentrisch und bevorzugt getrennt von dieser angeordnet ist, wobei eine Kulissenführung vorgesehen ist, die im Wirkzusammenhang mit dem Kulissenstein steht. Eine Bewegung der beweglichen Griffkomponente relativ zu der Basis über eine erste Strecke eines ersten Abschnitts der Kulissenführung überführt den Kulissenstein aus einer ersten Stellung, die zu der Schutzposition der Einführeinrichtung bzw. Nadel entspricht, in eine zweite Stellung, die der Einführposition der Einführeinrichtung korrespondiert. Diese Ausbildung ermöglicht eine relativ komplexe Führungsstrecke für die Einführeinrichtung beim Herausschwenken aus der Aufnahme, wobei gleichzeitig relativ geringe Kräfte über die bewegliche Griffkomponente ausgeübt werden müssen und ein Verkanten zwischen den einzelnen Bestandteilen der Mechanik höchst unwahrscheinlich ist.

Gemäß einer anderen vorteilhaften Ausführungsform der Erfindung kann ein Verbindungselement vorgesehen sein, das die Basis reversibel mit dem Griff verbindet, wobei zum Lösen des Griffs von der Basis der Griff über eine zweite Strecke senkrecht zur Erstreckungsrichtung der in der Einführposition befindlichen Einführeinrichtung bewegbar ist, wobei die Kulissenführung einen zu der zweiten Strecke korrespondierenden zweiten Abschnitt aufweist, so dass die Einführeinrichtung beim Lösen nicht mitbewegt wird. D. h., durch die passende Ausbildung der Kulissenführung ist es möglich, nach dem Einstechen der Einstechnadel und der Einführeinrichtung in den Körper eines Patienten eine Bewegung des Griffes parallel zur Körperoberfläche bzw. zur Oberfläche der Lasche, nachfolgend auch als Pflaster bezeichnet, zu bewerkstelligen, ohne dass dabei das organische Gewebe durch parallele Kräfte strapaziert wird. Auf diese Weise ist eine Trennung des Griffbereichs von der Basis und damit der Einstechnadel von der Einführeinrichtung in vorteilhafter Weise durch Betätigen derselben Komponente möglich, und hier insbesondere der beweglichen Griffkomponente, um sowohl die Auslösung als auch die Entkopplung vorzunehmen.

Es ist auch möglich, die Auslösung und Entkopplung unmittelbar hintereinander durchzuführen. Dabei durchläuft der Kulissenstein bereits die gesamte Kulissenführung, wodurch die Kanüle bzw. Einführeinrichtung in Verbindung mit der Einstichnadel bereits ausgeschwenkt wird, und gleichzeitig oder unmittelbar anschließend, die Entkopplung der Griffkomponenten durchgeführt wird, bevor die Kanüle mit der Nadel appliziert wird. Hier besteht dann nur noch ein leicht aufhebbarer Zusammenhalt zwischen dem im bzw. am Körper des Patienten verbleibenden Abschnitts und dem zu entfernenden Abschnitt des erfindungsgemäßen Insertionskopfes.

Vorteilhafterweise kann der Einführeinrichtung eine Sicherungsstruktur zugeordnet sein, die die Einführeinrichtung in der Schutzposition reversibel zurückhält. So kann beispielsweise die weitere Griffkomponente mit einer Rastschulter ausgebildet sein, die in einen reversiblen Eingriff mit einem komplementären Rastorgan an der beweglichen Griffkomponente bringbar ist. Wird ein Widerstand überwunden, kann eine Relativbewegung der weiteren gegenüber der beweglichen Griffkomponente erfolgen und die Kanüle nebst Einstechnadel kann in die Applikationsstellung verfahren oder geschwenkt werden.

Ferner kann in vorteilhafter Weise der Einführeinrichtung ein Kanülengehäuse zugeordnet sein, das mit der Einführeinrichtung mitbewegt wird, wobei das Kanülengehäuse die Sicherungsstruktur umfasst. Diese sollte einer parallel zu der Bewegungsrichtung der beweglichen Griffkomponente erstreckten Wand der beweglichen Griffkomponente gegenüberliegen. Dadurch wird es einem Eingriffselement an der Innenseite der Wand mit der Sicherungsstruktur ermöglicht, in der Schutzposition für die Einführeinrichtung im reversiblen Eingriff zu stehen. Mittels dieser Maßnahmen ist es möglich, die Einführeinrichtung in Verbindung mit der Einstechnadel sicher in der Schutzposition in der Aufnahme zu halten, um anschließend mit der beweglichen Griffkomponente dennoch nach Überwindung eines anfänglichen Widerstandes, der durch die Sicherungsstruktur und das Eingriffselement bereitgestellt wird, die gewünschte Schwenkbetätigung für die Einführeinrichtung in Verbindung mit der Einstechnadel bewerkstelligen zu können.

Gemäß einer weitere bevorzugten Ausführungsform ist es möglich, den erfindungsgemäßen Insertionskopf in der weiteren Griffkomponente, die die Einführeinrichtung nebst Einstechnadel oder diese allein in der Schutzposition aufnimmt, eine Sicherungskulisse vorzusehen, in der die Einführeinrichtung ggf. in Verbindung mit der Einstechnadel oder die Einstechnadel allein in der Schutzposition in der Aufnahme einrastbar ist. Durch die federnde Eigenschaft der Einführeinrichtung in Verbindung mit der Einstechnadel kann die Spitze der Einstechnadel entlang der Sicherungskulisse beim Zurückschwenken in die Schutzposition bzw. in die Aufnahme elastisch verformt bzw. seitlich ausgelenkt werden, um anschließend in eine Sicherungsausnehmung hinter einer Sicherungsschulter einzurasten. In dieser Position wird dann die Einstechnadel in Verbindung mit der Einführeinrichtung sicher in der Schutzposition gehalten. Die Sicherungskulisse kann auch in der Aufnahme oder der beweglichen Griffkomponente bzw. abschnittsweise an verschiedenen Bestandteilen vorgesehen sein.

Gemäß einer weiteren bevorzugten Ausführungsform kann die beweglichen Griffkomponente in die weitere Griffkomponente so weit einschiebbar sein, dass eine Steuerrampe an der beweglichen Griffkomponente nach Beendigung des Vorgangs zur Überführung der Einführeinrichtung von der Schutzposition in die Einführposition gegen eine Auslenkrampe an einem Verbindungselement, das die Basis reversibel mit dem Griff verbindet, anlaufen, um eine Verbindungseinrichtung aus ihrer Eingriffsstellung auszulenken, so dass der Griff und die Basis trennbar sind. Diese Ausbildung insbesondere in Verbindung mit der zweiten Strecke der Kulissenführung ist besonders vorteilhaft, um ohne Beanspruchung des Gewebes des Patienten den erfindungsgemäßen Insertionskopf von der Einstechfunktion in die Zuführfunktion zum Zuführen eines Medikaments zu überführen.

Vorteilhafterweise ist sowohl die Basis mit de Kanülengehäuse als auch der Nadelhalter mit den zweiten oder den ersten Griffkomponenten drehbar bzw. schwenkbar verbunden. Dabei weisen die beiden Dreh- bzw. Schwenkmechaniken bevorzugt ein gemeinsames Zentrum bzw. eine gemeinsame Achse auf.

Damit ist es möglich, gemäß der Erfindung die kontaminierte Einstecheinrichtung bzw. Einstechnadel wieder in die zweite Griffkomponente zurückgeschwenkt werden.

Bevorzugte Merkmale werden auch in den Unteransprüchen und deren Kombinationen offenbart.

Nachfolgend werden Ausführungsbeispiele der Erfindung anhand von Figuren erläutert. An den Ausführungsbeispielen offenbar werdende Merkmale bilden je einzeln und in jeder Merkmalskombination die Gegenstände der Ansprüche und auch die vorstehend beschriebenen Ausgestaltungen vorteilhaft weiter. Es zeigen:
Figur 1 einen Insertionskopf eines ersten Ausführungsbeispiels mit in Schutzposition befindlicher Einführeinrichtung,
Figur 2 den Insertionskopf mit in Einführposition befindlicher Einführeinrichtung,
Figur 3 einen Griff des Insertionskopfs des ersten Ausführungsbeispiels,
Figur 4 eine Basis des Insertionskopfs des ersten Ausführungsbeispiels mit in Einführposition befindlicher Einführeinrichtung,
Figur 5 den Griff der Figur 3 in einer Ansicht
Figur 6 die Basis mit Einführeinrichtung der Figur 4 in einer Ansicht,
Figur 7 einen Insertionskopf eines zweiten Ausführungsbeispiels mit in Schutzposition befindlicher Einführeinrichtung,
Figur 8 den Insertionskopf des zweiten Ausführungsbeispiels mit in Einführposition befindlicher Einführeinrichtung,
Figur 9 einen Griff des Insertionskopfs des zweiten Ausführungsbeispiels,
Figur 10 eine Basis des Insertionskopfs des zweiten Ausführungsbeispiels mit in Einführposition befindlicher Einführeinrichtung,
Figur 11 ein System aus einem Insertionskopf und einem als solchen nicht beanspruchten Inserter eines ersten Ausführungsbeispiels vor einer Aktivierung,
Figur 12 das System des ersten Ausführungsbeispiels nach der Aktivierung,
Figur 13 ein System aus einem Insertionskopf und einem als solchen nicht beanspruchten Inserter eines zweiten Ausführungsbeispiels vor einer Aktivierung des Insertionskopfs,
Figur 14 das System des zweiten Ausführungsbeispiels nach der Aktivierung und
Figur 15 das System des zweiten Ausführungsbeispiels nach einer Platzierung des Inse-rtionskopfs auf einer Gewebeoberfläche.
Fig. 16 einen Insertionskopf mit in Schutzposition befindlicher Einführeinrichtung in einer Seitenansicht,
Fig. 17 den Insertionskopf nach Fig. 16 mit in Einführposition befindlicher Einführeinrichtung,
Fig. 18 den Insertionskopf nach den Fig. 16 und 17, wobei der Griff nebst Einstechnadel von der Basis nebst Einführeinrichtung getrennt ist,
Fig. 19 die weitere und die bewegliche Griffkomponente im von der Basis gelösten Zustand in einer Seitenansicht,
Fig. 20 einen Insertionskopf mit in Schutzposition befindlicher Einführeinrichtung in einer Schnittansicht,
Fig. 20a einen Ausschnitt aus Fig. 20 ebenfalls in einer Schnittdarstellung,
Fig. 21 den Insertionskopf gemäß Fig. 20 mit in Einführposition befindlicher Einführeinrichtung, ebenfalls in einer Schnittdarstellung,
Fig. 22 in einer Schnittdarstellung den Griff nebst Einstichabschnitt getrennt von der Basis mit Einführeinrichtung, die am Patienten platziert sein kann,
Fig. 23 in einer Schnittdarstellung die weitere und die bewegliche Griffkomponente nach dem Herausziehen der beweglichen Griffkomponente aus der weiteren Griffkomponente, nebst einem vergrößerten Ausschnitt C ebenfalls in Schnittdarstellung,
Fig. 23a einen Schnitt D-D gemäß Fig. 23, der senkrecht zu dem Schnitt gemäß Fig. 23 angelegt ist,
Fig. 24 einen Insertionskopf gemäß der Erfindung in einer perspektivischen Ansicht vor der Applikation,
Fig. 25 den Insertionskopf gemäß Fig. 24 nach der Applikation,
Fig. 26 den Insertionskopf gemäß den Fig. 24 und 25 nach Entfernung der weiteren und beweglichen Griffkomponenten nebst Einstechnadel,
Fig: 27 die weitere und bewegliche Griffkomponente in einer perspektivischen Ansicht nach dem Herausziehen der beweglichen Griffkomponente aus der weiteren Griffkomponente,
Fig. 28 die Basis des Insertionskopfes nach den Fig. 24 bis 27 nach dem Andocken eines Konnektors zur Zuführung eines Medikaments,
Fig. 29 einen Insertionskopf mit in Schutzposition befindlicher Einführeinrichtung nebst Einstechnadel, dargestellt ohne die weitere Griffkomponente,
Fig. 30 Insertionskopf gemäß Fig. 29 mit Einführeinrichtung nebst Einstechnadel in Applikationsposition,
Fig. 31 einen Insertionskopf mit in Schutzposition befindlicher Einführeinrichtung in einer Seitenansicht, und
Fig. 32 einen Schnitt E-E durch den Insertionskopf gemäß Fig. 31 in einer Draufsicht.

Nachfolgend werden gleiche oder zumindest funktionsgleiche Bestandteile in der Regel mit gleichen oder vergleichbaren Bezugszeichen benannt, so dass eine mehrfache Beschreibung meist unterbleiben kann. Die Bestandteile der einzelnen Ausführungsformen können größtenteils miteinander ausgetauscht werden, d.h., kombiniert werden. Aus den nachfolgend beschriebenen Ausführungen gehen weitere Merkmale, Zielsetzungen sowie Vorteile der Erfindung hervor.

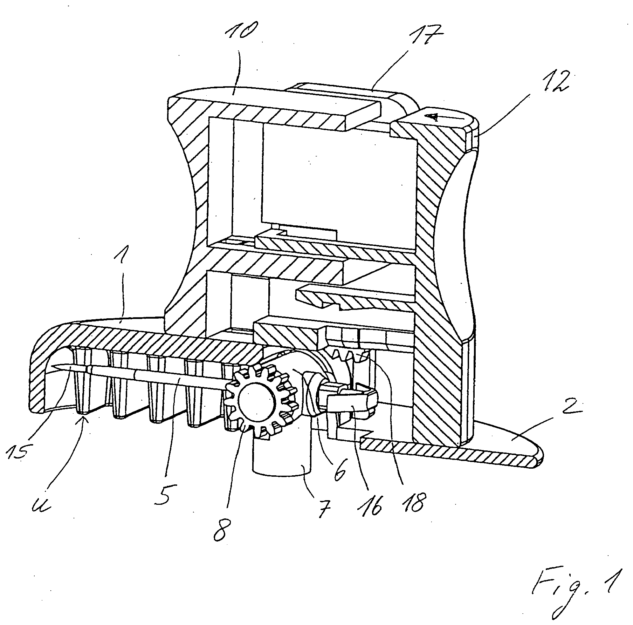

Figur 1 zeigt einen Insertionskopf eines ersten Ausführungsbeispiels in einem Längsschnitt. Der Insertionskopf umfasst eine Basis mit einem Aufnahmeteil bzw. einer Aufnahme 1 und einem Flachteil 2, die in einem Stück aus Kunststoff geformt sind. Die Basis 1, 2 ist mit ihrer Unterseite U auf organischem Gewebe platzierbar. Der Insertionskopf umfasst des Weiteren einen zweiteiligen Griff mit einer ersten Griffkomponente 10 und einer zweiten Griffkomponente 12. Die Griffkomponente 10 ist mit der Basis unbeweglich, aber lösbar verbunden. Die Griffkomponente 12 ist an der Griffkomponente 10 beweglich gehalten, wobei die Griffkomponente 12 sowohl relativ zu der Griffkomponente 10 als auch zu der Basis 1, 2 linear verschiebbar ist. Die Achse der Bewegbarkeit der Griffkomponente 12 weist parallel zu einer Unterseite U der Basis 1, 2. Die Richtung der Bewegbarkeit ist auf der Oberseite der Griffkomponente 12 mit einem Pfeil angedeutet.

Die Basis 1, 2 lagert eine Einführeinrichtung 5 schwenkbeweglich um eine zur Unterseite U parallele Rotationsachse. Die Einführeinrichtung 5 ist lang gestreckt. Im Ausführungsbeispiel ist sie als flexible Kanüle gebildet. Die Einführeinrichtung 5 wird von einer Einstecheinrichtung 15 durchragt, die als dünne Nadel gebildet ist, deren Biegesteifigkeit ausreicht, um die Einstecheinrichtung 15 gemeinsam mit der umgebenden, sich anschmiegenden Einführeinrichtung 5 durch die Hautoberfläche in subkutanes Gewebe einzustechen und dadurch die Einführeinrichtung 5 einzuführen. In bevorzugten Ausführungen ist an der Unterseite U ein Klebepad für eine Fixierung des Insertionskopfs auf dem Gewebe, vorzugsweise der Hautoberfläche, angebracht.

Für die Schwenkbeweglichkeit der Einführeinrichtung 5 und damit gemeinsam der Einstecheinrichtung 15 sorgt ein Gelenkelement 6, das eine Welle eines Drehgelenks mit der Rotationsachse als Gelenkachse bildet. Die Basis 1, 2 bildet das andere Gelenkelement des Drehgelenks in Form einer Buchse oder gegebenenfalls auch eines offenen Lagerauges. Auf der Rotationsachse des Gelenks ist zu beiden Seiten des Gelenkelements 6 je ein außenverzahntes Zahnrad 8 angeordnet und drehsteif mit dem Gelenkelement 6 verbunden, beispielsweise in einem Stück geformt. Das eine der beiden Zahnräder 8 ist in Figur 1 erkennbar. Das andere sitzt auf der gegenüberliegenden Seite des Gelenkelements 6 und wird von dem Aufnahmeteil 1 der Basis 1, 2 verdeckt. Die Einstecheinrichtung 15 durchragt das Gelenkelement 6. An das Gelenkelement 6 schließt sich eine Zuführung 7 für eine Medikamentenflüssigkeit, beispielsweise Insulin, an. Die Zuführung 7 ragt in etwa rechtwinklig zur Einführeinrichtung 5 von dem Gelenkelement 6 ab. Das Gelenkelement 6 bildet mit der Zuführung 7, den Zahnrädern 8, der Einführeinrichtung 5 und der Einstecheinrichtung 15 eine in Bezug auf die Drehbewegung des Gelenkelements 6 und der Zahnräder 8 und der Schwenkbewegung der genannten weiteren Komponenten eine Einheit.

Die bewegliche Griffkomponente 12 ist mit zwei Zahnstangen 18 versehen, die je mit einem der Zahnräder 8 in Zahneingriff sind. Von den beiden Zahnstangen 18 ist nur die mit dem verdeckten Zahnrad zusammenwirkende erkennbar. Eine ebensolche Zahnstange 18 wirkt mit dem erkennbaren Zahnrad 8 zusammen. Im Falle einer Verschiebung der Griffkomponente 12 in die durch den Richtungspfeil angedeutete Richtung, wobei die erste Griffkomponente 10 bei dieser Bewegung die Griffkomponente 12 führt, kämmen die beiden Zahnstangen 18 mit den beiden Zahnrädern 8, so dass die Verschiebebewegung der Griffkomponente 12 in eine Drehbewegung des Gelenkelements 6 und eine Schwenkbewegung der Einführeinrichtung 5 und der Einstecheinrichtung 15 sowie der Zuführung 7 übertragen wird.

Die Schwenkbewegung überführt die Einführeinrichtung 5 aus deren in Figur 1 gezeigten Schutzposition in eine Einführposition. In der Schutzposition weisen die Einführeinrichtung 5 und die Einstecheinrichtung 15 zumindest im Wesentlichen parallel zu der Unterseite U der Basis 1, 2. Die Einführeinrichtung 5 und der in die gleiche Richtung über das Gelenkelement 6 hinausstehende Abschnitt der Einstecheinrichtung 15 sind in der gemeinsamen Schutzposition in einem von dem Aufnahmeteil bzw. der Aufnahme 1 mit Ausnahme der Unterseite U umschlossenen Hohlraum aufgenommen. Mit in Schutzposition befindlicher Einführeinrichtung 5 ist gewährleistet, dass der Benutzer sich nicht an der Einstecheinrichtung 15 verletzen und umgekehrt die Einführeinrichtung 5 und die Einstecheinrichtung 15 nicht durch unachtsame Handhabung beschädigt werden können. Die Aufnahme 1 bildet bevorzugt auch einen Sichtschutz, so dass der Benutzer die Einstecheinrichtung 15 von der Oberseite des Insertionskopfs und auch bei seitlichem Blickwinkel nicht erkennen kann. Ein an der Unterseite bevorzugterweise angebrachtes Klebepad ist mit einem Durchtrittsschlitz für die Einführeinrichtung 5 und die Einstecheinrichtung 15 versehen.

Die Zahnstangen 18 sind jeweils an einer der Unterseite U zugewandten Unterseite zweier biegesteifer Zungen geformt, die von einem Seitenteil der Griffkomponente 12 in Bewegungsrichtung vorragen. Über die beiden die Zahnstangen 18 bildenden Zungen hinaus ragt von dem Seitenteil der Griffkomponente 12 wenigstens eine weitere Zunge in Bewegungsrichtung vor, die der Linearführung der beweglichen Griffkomponente 12 an einer von der Griffkomponente 10 gebildeten Führung dient.

Um die Einführeinrichtung 5 in das Körpergewebe bis unter oder gegebenenfalls nur in die Haut einzuführen, greift der Benutzer den Insertionskopf am Griff zwischen Daumen und Zeigefinger. Die Griffkomponenten 10 und 12 sind je mit einer entsprechend geformten, seitlichen Einbuchtung versehen. Durch Zusammendrücken der Griffkomponenten 10 und 12 wird die bewegliche Griffkomponente 12 bis gegen einen von der ersten Griffkomponente 10 gebildeten Anschlag gedrückt. Bei dieser Bewegung kämmen die beiden Zahnstangen 18 mit den Zahnrädern 8, so dass die Translationsbewegung der Griffkomponente 12 in eine Drehbewegung des Gelenkelements 6 und somit in eine Schwenkbewegung der Einführeinrichtung 5 und der Einstecheinrichtung 15 übertragen wird. Der Weg der Griffkomponente 12, der Durchmesser der Zahnräder 8 und die Feinheit der Verzahnungen sind so gewählt, dass eine Bewegung der Griffkomponente 12 um wenige Millimeter, beispielsweise 4 oder 5 mm, eine Schwenkbewegung der Einführeinrichtung und der Einstecheinrichtung 15 um einen Schwenkwinkel von zumindest im wesentlichen 90° in eine Einführposition bewirkt, in der die Einführeinrichtung 5 und die Einstecheinrichtung 15 über die Unterseite U der Basis 1, 2 zumindest in etwa rechtwinklig vorragen.

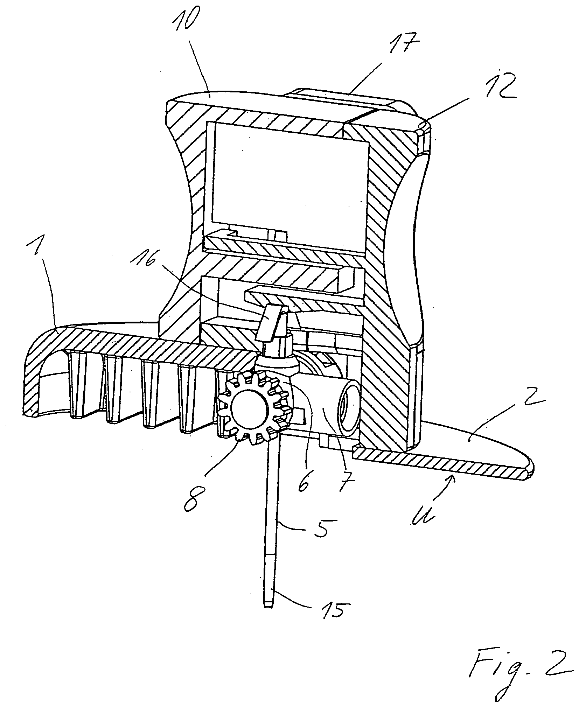

Figur 2 zeigt den Insertionskopf mit der in Einführposition befindlichen Einführ- und Einstecheinrichtung 5, 15.

Die Einstecheinrichtung 15 hat sich am Ende der Schwenkbewegung mit dem Griff 10, 12 verbunden, im Ausführungsbeispiel mit der beweglichen Griffkomponente 12. In der Schutzposition (Figur 1) besteht zwischen der Einstecheinrichtung 15 und dem Griff 10, 12 kein Kontakt, so dass die Einstecheinrichtung 15 gemeinsam mit der Einführeinrichtung 5 frei schwenken kann. Zur Herstellung der Verbindung ist an einem in der Einführposition proximalen Ende der Einstecheinrichtung 15, mit dem die Einstecheinrichtung 15 über das Gelenkelement 6 hinaussteht, ein Verbindungselement 16 angeordnet, im Ausführungsbeispiel befestigt. Das Verbindungselement 16 weist einen oder zwei abstehende Flügel auf, mit denen es ein Verbindungsgegenelement der Griffkomponente 12 in Bezug auf die Längsrichtung der Einstecheinrichtung 15 hintergreift. Das Verbindungsgegenelement der beweglichen Griffkomponente 12 ist als entsprechend hintergreifbare Schulterfläche gebildet.

Für die Platzierung des Insertionskopfs auf einer Gewebeoberfläche und der Einführung der Einführeinrichtung 5 in das Gewebe hält der Benutzer den Insertionskopf am Griff 10, 12 und bewegt ihn auf die Gewebeoberfläche zu. Dabei durchsticht die Einstecheinrichtung 15 die Gewebeoberfläche, vorzugsweise die menschliche Haut, und dringt in die Haut ein. Die sich anschmiegende Einführeinrichtung 5 dringt gemeinsam mit der Einstecheinrichtung 15 ein, bis der Insertionskopf mit seiner Unterseite U auf der Gewebeoberfläche aufsetzt und sich dabei adhäsiv auf der Hautoberfläche fixiert, vorzugsweise mittels eines Klebepads. Für die Verabreichung des Medikaments wird die Einstecheinrichtung 15 entfernt und die Zuführung 7 über einen mit der Zuführung 7 zusammenwirkenden Konnektor mit einem Medikamentenreservoir, vorzugsweise einer Medikamentenpumpe, verbunden. Um dies zu ermöglichen, wird zuvor der Griff 10, 12 von der Basis 1, 2 gelöst. Das Lösen ist nur nach dem Zusammendrücken bzw. Zusammenschieben der Griffkomponenten 10 und 12 möglich. Allerdings löst sich die Verbindung bei der Bewegung der Griffkomponente 12 automatisch, so dass der Griff 10, 12 in die proximale Richtung, in Figur 2 nach oben, abgezogen werden kann. Bei der geraden Abziehbewegung gleitet die Einstecheinrichtung 15 durch die Einführeinrichtung 5 und das Gelenkelement 6 und gibt dadurch den Strömungsquerschnitt der Einführeinrichtung 5 frei, so dass der Strömungsquerschnitt nach Herausziehen der Einstecheinrichtung 15 auch gleichzeitig mit der Zuführung 7 fluidisch verbunden ist. Diesbezüglich kann der Insertionskopf wie beispielsweise in der DE 198 21 723 C1 und der DE 10 2004 039 408.3 beschrieben ausgebildet sein.

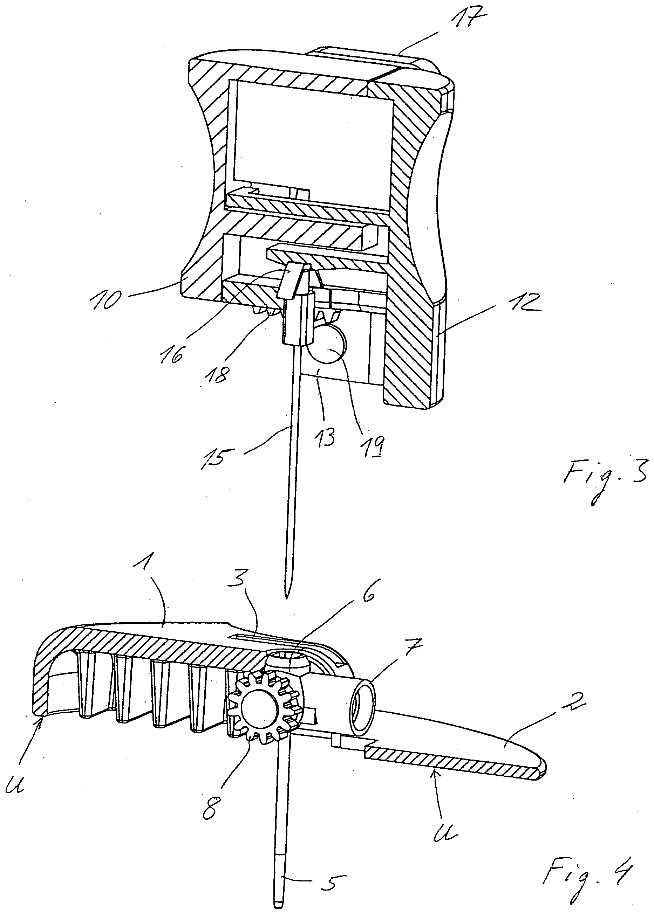

Die Figuren 3 und 4 zeigen die beiden voneinander gelösten Teile des Insertionskopfs, nämlich die Basis 1, 2 mit der Einführeinrichtung 5 einerseits und den Griff 10, 12 mit der Einstecheinrichtung 15 andererseits, in zueinander ausgerichteter Position, in der die Längsachse der Einführeinrichtung 5 und die Längsachse der Einstecheinrichtung 15 miteinander fluchten. In Figur 3 ist auch eine Ausnehmung 3 in der Basis 1, 2 erkennbar, in die im verbundenen Zustand bei Bewegung der Griffkomponente 12 eine der beiden Zahnstangen 18 einfährt und dabei mit dem in der Ausnehmung 3 angeordneten Zahnrad 8 kämmt. Die Ausnehmung 3 ist schlitzförmig. Ferner ist ein Verbindungselement 19 des Griffs 10, 12 erkennbar, das im verbundenen Zustand formschlüssig in ein Verbindungsgegenelement der Basis 1, 2 eingreift und so den Griff 10, 12 an der Basis 1, 2 hält und in Kombination mit Kontaktflächen des Griffs 10, 12 und der Basis 1, 2 relativ zu der Basis 1, 2 fixiert. Das Verbindungselement 19 ragt stummelartig von einer im distalen Bereich von der Griffkomponente 10 abragenden, elastischen Lasche 13 in eine zur Unterseite U der Basis 1, 2 parallel weisende Richtung vor und im verbundenen Zustand in eine Ausnehmung der Basis 1, 2 hinein, beispielsweise ein zum Verbindungselement 19 kongruent geformtes Loch, so dass eine Bewegung des Griffs 10, 12 in Längsrichtung des Einstechabschnitts 15 bei bestehender Verbindung verhindert wird. Um diese Verbindung zu lösen, ragt von dem Seitenteil der beweglichen Griffkomponente 12 eine nicht dargestellte weitere Zunge vor, die bei der Bewegung der Griffkomponente 12 zwischen die Basis 1, 2 und die das Verbindungselement 19 tragende Lasche 13 fährt und die Lasche 13 von der Basis 1, 2 ein Stück weit abbiegt, das jedoch ausreicht, die formschlüssige Verbindung zwischen dem Verbindungselement 19 und dem Verbindungsgegenelement zu lösen, so dass der Griff 10, 12 mit der Einstecheinrichtung 15 in deren Längsrichtung von der Basis 1, 2 abgezogen werden kann.

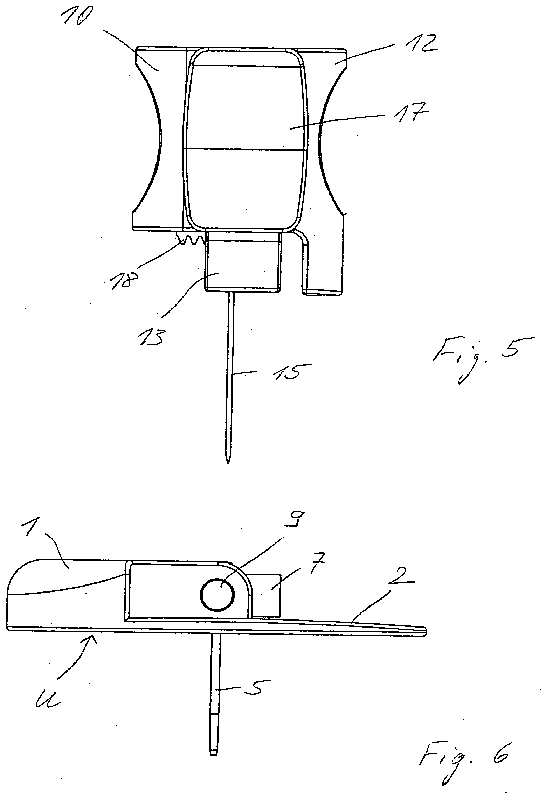

Die Figuren 5 und 6 zeigen nochmals die voneinander gelösten Teile des Insertionskopfs in einer Ansicht auf die in den Figuren 1 bis 4 abgewandte Rückseite. In dieser Ansicht ist in Figur 6 insbesondere das Verbindungsgegenelement 9 der Basis 1, 2 erkennbar, das im verbundenen Zustand, d.h. im Eingriff mit dem Verbindungselement 19, den Griff 10, 12 an der Basis 1, 2 hält.

Die in den Figuren 4 und 6 einzeln dargestellte Basis 1, 2, die Träger der Einführeinrichtung 5 und der damit eine Schwenkeinheit bildenden Teile 6, 7 und 8 ist, verbleibt auf der Gewebeoberfläche und ist in diesem Sinne ein Verbleibteil. Der Griff 10, 12 hingegen, der nun als Träger für die Einstecheinrichtung 15 dient, wird entsorgt oder gegebenenfalls wieder von der Einstecheinrichtung 15 gelöst und einer erneuten Verwendung zugeführt, während die Einstecheinrichtung 15 entsorgt wird. Das Verbleibteil 1-9 kann somit vorteilhaft flach sein und stört nicht, wenn es unter der Kleidung getragen wird. Die Flexibilität der Einführeinrichtung 5 ist derart, dass die Einführeinrichtung 5 im eingeführten Zustand als nicht störend empfunden wird, aber doch ausreichend stabil ist, um eine Versorgung mit dem Medikament sicher zu gewährleisten.

Der Griff 10, 12 kann auch in solchen Ausführungen des Insertionskopfs verwendet werden, in denen die Einführeinrichtung nicht wie die Einführeinrichtung 5 von Hause aus flexibel ist, sondern ohne Fremdstabilisierung für das Einstechen ausreichend biegesteif ist. In derartigen Ausführungen kann die zusätzliche Einstecheinrichtung 15 entfallen. Der Griff 10, 12 dient in derartigen Ausführungen ausschließlich der Handhabung des Insertionskopfs, nicht jedoch als Träger einer stabilisierenden Einstecheinrichtung 15. Eine derart modifizierte Einführeinrichtung 5 kann insbesondere als Einstechkanüle mit einem Hohlquerschnitt oder als Einstechnadel mit einem vollen Querschnitt und einem oder mehreren Strömungskanälen am äußeren Umfang gebildet sein, die nach der Einführung durch Wechselwirkung mit dem Gewebe flexibler wird.

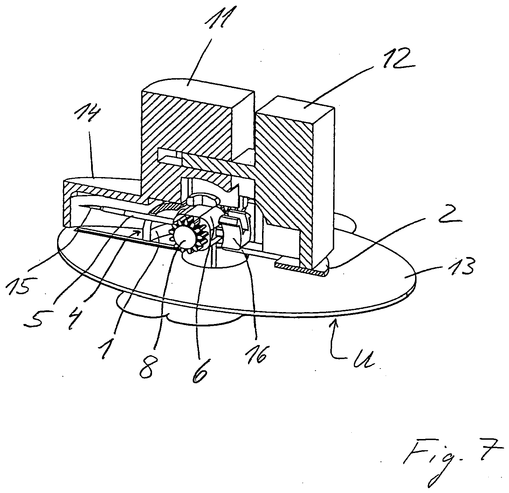

Die Figuren 7 bis 10 zeigen ein zweites Ausführungsbeispiel eines Insertionskopfs. Von den nachfolgend beschriebenen Unterschieden abgesehen entspricht der Insertionskopf des zweiten Ausführungsbeispiels dem Insertionskopf des ersten Ausführungsbeispiels.

So ist beispielhaft ein an der Unterseite U befestigtes Klebepad dargestellt, wie es auch an dem Insertionskopf des ersten Ausführungsbeispiels angebracht sein könnte.

Gegenüber dem ersten Ausführungsbeispiel sind die Aufnahme 1 und die erste Griffkomponente 11 modifiziert. Im Unterschied zum ersten Ausführungsbeispiel sind die Einführeinrichtung 5 und die Einstecheinrichtung 15 nur über einen kurzen Abschnitt in der vom Aufnahmeteil gebildeten Aufnahme 1 aufgenommen. Im zweiten Ausführungsbeispiel bildet der Griff, genauer gesagt dessen Griffkomponente 11, eine Aufnahme 14 für die Einführeinrichtung 5 und die Einstecheinrichtung 15. Die Aufnahme 1 ist seitlich mit einer zur Unterseite U offenen Ausnehmung 4 in Form eines Schlitzes versehen, durch welche die Einführeinrichtung 5 und die Einstecheinrichtung 15 in der Schutzposition aus der Aufnahme 1 hinausragen. Die Aufnahme 1 ist ihrerseits in der Aufnahme 14 aufgenommen. Die Aufnahme 14 ist zur Unterseite U hin offen, umschließt aber ansonsten die Einführeinrichtung 5 und die Einstecheinrichtung 15, vorzugsweise blickdicht.

Um die Einführeinrichtung 5 und die Einstecheinrichtung 15 aus der Schutzposition in die Einführposition zu schwenken, führt der Benutzer die anhand des ersten Ausführungsbeispiels beschriebenen Handgriffe durch, d.h. er drückt die bewegliche Griffkomponente 12 gegen die modifizierte Griffkomponente 11. Bei dem kämmenden Zahneingriff schwenken die Einführeinrichtung 5 und Einstecheinrichtung 15 in der Ausnehmung 4 aus der Aufnahme 1 und insbesondere aus der Aufnahme 14 in die Einführposition.

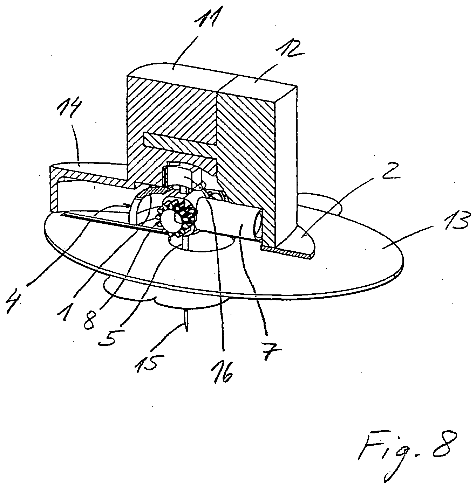

Figur 8 zeigt den Insertionskopf des zweiten Ausführungsbeispiels mit in Einführposition befindlicher Einführeinrichtung 5 und Einstecheinrichtung 15.

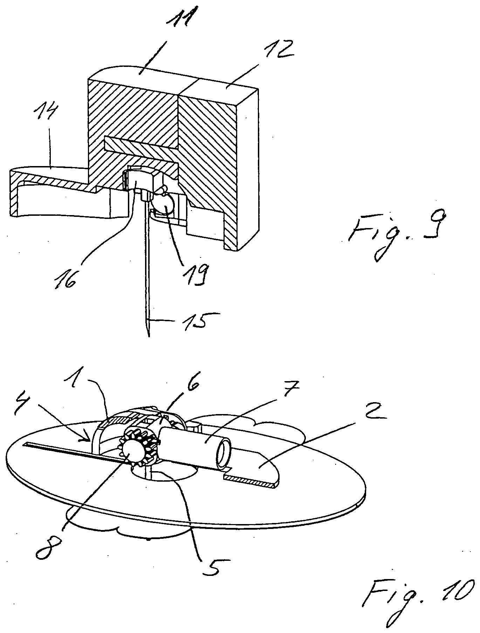

Die Figuren 9 und 10 entsprechend bezüglich des zweiten Ausführungsbeispiels den Figuren 3 und 4 zum ersten Ausführungsbeispiels. Wie jedoch nicht zuletzt der Blick auf Figur 10 zeigt, ist die Basis 1, 2 des zweiten Ausführungsbeispiels gegenüber der Basis 1, 2 des ersten Ausführungsbeispiels vorteilhaft verkürzt, da nicht mehr sie, sondern der Griff 11, 12 für die Einführeinrichtung 5 und die Einstecheinrichtung 15 in deren Schutzposition die Schutzfunktion übernimmt.

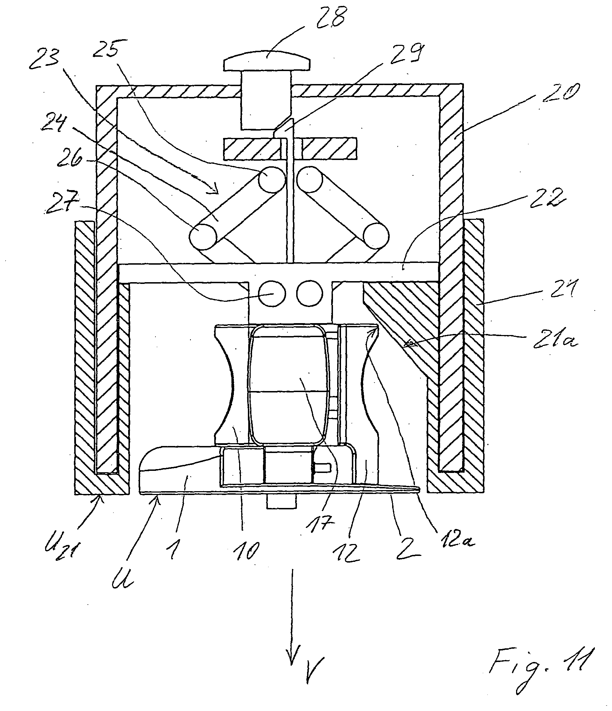

Figur 11 zeigt ein System eines ersten Ausführungsbeispiels bestehend aus dem Insertionskopf des ersten Ausführungsbeispiels und einem Inserter, welcher der Platzierung des Insertionskopfs auf dem Gewebe dient, so dass der Benutzer den Insertionskopf bei der Platzierung nicht zwischen den Fingern greifen muss. Insbesondere hält der Benutzer bei der Überführung der Einführeinrichtung 5 in die Einführposition den Insertionskopf nicht am Griff. Diese Aktivierung des Insertionskopfs wird mit Hilfe des Inserters vorgenommen. Der Benutzer wird daher noch sicherer vor Stichverletzungen und die Einführeinrichtung 5 sowie die Einstecheinrichtung 15 werden noch sicherer vor Beschädigungen durch unachtsame Handhabung geschützt, nämlich durch den Inserter.

Der Inserter weist ein Insertergehäuse 20 auf, das als Hülsenteil mit einem Boden gebildet ist und von außen betrachtet im Wesentlichen die Form eines Topfs aufweist. Das Insertergehäuse 20 nimmt eine Halteeinrichtung und einen Antrieb für den Insertionskopf auf. Die Halteeinrichtung umfasst eine Haltefeder, beispielsweise eine Blattfeder, die den Insertionskopf in der in Figur 11 gezeigten Ausgangsposition relativ zu dem Insertergehäuse 20 hält. In dem Halteeingriff hintergreift die Haltefeder eine an dem Griff 10, 12 geformte Haltestruktur 17, die in den Figuren 1, 2, 3 und 5 erkennbar ist. Der Halteeingriff ist gegen die rückstellende Elastizitätskraft der Haltefeder lösbar.

Der Antrieb umfasst ein Vortriebselement 22, das in und gegen eine Vortriebsrichtung V linear beweglich in dem Insertergehäuse 20 angeordnet ist. Die Vortriebsrichtung V fällt mit einer zentralen Längsachse des Insertergehäuses 20 zusammen. Der Antrieb umfasst ferner einen Krafterzeuger 23, der in die Vortriebsrichtung V auf das Vortriebselement 22 wirkt. Der Krafterzeuger 23 umfasst zwei Paare von gelenkig miteinander verbundenen Schenkeln 24, wobei die beiden Paare von Schenkeln 24 symmetrisch bezüglich der zentralen Längsachse, d. h. symmetrisch zur Vortriebsrichtung V, des Insertergehäuses 20 angeordnet sind. Jedes der Schenkelpaare ist in einem zum Insertergehäuse 20 ortsfesten Drehgelenk 25 aufgehängt. Die beiden Schenkel 24 des jeweiligen Schenkelpaars sind in einem freien Drehgelenk 26 miteinander drehbeweglich verbunden. Ferner ist der von dem ortsfesten Gelenk 25 abgewandte Schenkel 24 jeweils in einem Drehgelenk 27 mit dem Vortriebselement 22 verbunden. Nicht dargestellte Federn oder gegebenenfalls auch nur eine Feder spannt diese Schenkel-Gelenk-Vortriebselement-Anordnung in die Vortriebsrichtung V. Die Anordnung aus Schenkeln 24 und Gelenken 25, 26 und 27 führt das Vortriebselement 22; zusätzlich oder stattdessen könnte die Mantelinnenfläche des Insertionsgehäuses 23 das Vortriebselement 22 führen. Ferner ist ein Blockierglied 29 vorgesehen, das mit dem Insertergehäuse 20 in einem Blockiereingriff ist, der eine Vortriebsbewegung des Vortriebselements 22 verhindert. Das Blockierglied 29 kann den Blockiereingriff mit der von dem Insertergehäuse 20 gebildeten Hüllstruktur oder ebenso mit einer damit in Bezug auf die Vortriebsrichtung V fest verbundenen sonstigen Struktur bilden. Der Blockiereingriff ist durch Betätigung eines druckknopfartigen Auslösers 28 lösbar.

Der Inserter umfasst ferner ein Aktivierungsglied 21, das mit dem Insertergehäuse 20 in und gegen die Vortriebsrichtung V beweglich verbunden ist. Das Aktivierungsglied 21 bildet in Bezug auf das Insertergehäuse 20 eine Muffe, so dass insgesamt ein zweiteiliges, teleskopierbares Insertergehäuse mit Gehäuseteilen 20 und 21 erhalten wird. Der Unterscheidung in funktionaler Hinsicht wegen wird das Gehäuseteil 21 jedoch weiterhin als Aktivierungsglied bezeichnet. Das Aktivierungsglied 21 bildet die Unterseite U₂₁ des Inserters, mit welcher der Inserter für die Platzierung des Insertionskopfs auf der Gewebeoberfläche aufsetzbar ist und vorzugsweise auch aufgesetzt wird. In der in Figur 11 vom Insertionskopf eingenommenen Ausgangsposition weisen die Unterseite U₂₁ des Inserters und die Unterseite U des gehaltenen Insertionskopfs jeweils in die Vortriebsrichtung V, die für die beiden Unterseiten zumindest im Wesentlichen eine Flächennormale bildet.

Das Aktivierungsglied 21 umfasst ein äußeres Hülsenteil und ein inneres Hülsenteil, die an der Unterseite U₂₁ miteinander verbunden sind und zwischen sich einen Ringspalt freilassen. Das Insertergehäuse 20 ragt in diesen Ringspalt und führt das Aktivierungsglied 21 gleitbeweglich.

In dem in Figur 11 gezeigten Zustand nimmt das Aktivierungsglied 21 relativ zu dem Insertergehäuse 20 eine eingefahrene Position ein und der Inserter weist eine in Vortriebsrichtung V gemessen kürzeste Länge auf. In diesem Zustand des Inserters wird der Insertionskopf eingesetzt, d. h. in den Halteeingriff mit der Halteeinrichtung des Inserters gebracht. Anstatt den Insertionskopf einzusetzen, kann der Inserter auch über den auf einer Auflage liegenden Insertionskopf gestülpt werden. Die Position und Geometrie der Halteeinrichtung ist so gewählt, dass der Halteeingriff bei dem Aufstülpen automatisch entsteht. Unmittelbar nach dem Aufnehmen des Insertionskopfs, beispielsweise durch Einsetzen, befindet sich die Einführeinrichtung 5 des Insertionskopfs in ihrer Schutzposition. In diesem Sinne ist der Insertionskopf noch inaktiv. Der Inserter ist mit Mitteln ausgestattet, nämlich dem Aktivierungsglied 21, durch deren Betätigung die Einführeinrichtung in die Einführposition bewegt und auf diese Weise der Insertionskopf aktiviert werden kann.

Für die Aktivierung bilden das Aktivierungsglied 21 und der Insertionskopf miteinander ein Gelenk, im Ausführungsbeispiel ein Kurvengelenk. Die beiden Gelenkelemente des Gelenks sind eine Führungskurve 21a, die das Aktivierungsglied 21 bildet, und ein von der beweglichen Griffkomponente 12 gebildetes Eingriffselement 12a. In der Kopplung, über die das Aktivierungsglied 21 auf die Einführeinrichtung 5 einwirkt, bildet die bewegliche Griffkomponente 12 ein Eingangs- bzw. Empfangsglied des Insertionskopfs. Wird das Aktivierungsglied 21 relativ zu dem Insertergehäuse 20 in die Vortriebsrichtung V bewegt, gleitet die Führungskurve 21a über das Eingriffselement 12a, d. h. über die das Eingriffselement 12a bildende Kontaktfläche des Empfangsglieds, d. h. der beweglichen Griffkomponente 12. Durch den Druckkontakt und den zur Vortriebsrichtung V geneigten Verlauf der Führungskurve 21a bewegt sich die Griffkomponente 12 quer zu der Vortriebsrichtung V auf die andere Griffkomponente 10 zu, und die Einführeinrichtung 5 schwenkt wie zum Insertionskopf als solchem beschrieben in die Einführposition. Die bewegliche Griffkomponente 12 bildet das Eingriffselement 12a an ihrem von der Basis 1, 2 abgewandten oberen Ende, im Ausführungsbeispiel mit ihrer äußeren Kante. Die Führungskurve 21a ist der Unterseite U₂₁ des Inserters zugewandt. Die Neigung ist so gewählt, dass sich die Führungskurve 21a von einem von der Unterseite U₂₁ abgewandten Ende in Vortriebsrichtung V von dem Insertionskopf bzw. der im aktivierten Zustand ausgeschwenkten Einführeinrichtung 5 oder der zentralen Längsachse des Inserters weg neigt. Der Neigungswinkel ist überall konstant, die Führungskurve 21a ist eine Schräge, d. h. eine schräge Linie oder Fläche.

Für die praktische Handhabung bietet es sich an, dass der Benutzer den Inserter nach dem Aufnehmen des Insertionskopfs mit der einen Hand am Aktivierungsglied 21 hält, beispielsweise indem er das Aktivierungsglied 21 umgreift, und mit der anderen Hand das Insertergehäuse 20 relativ zu dem gehaltenen Aktivierungsglied 21 gegen die Vortriebsrichtung V zieht. Auch dies wird als Betätigung des Aktivierungsglieds verstanden. Das Vortriebselement 22 und der Krafterzeuger 23 bewegen sich gemeinsam mit dem Insertergehäuse 20 relativ zu dem Aktivierungsglied 21. Der von der Halteeinrichtung in der Ausgangsposition gehaltene Insertionskopf wird mitgenommen, d. h. bewegt sich gleichermaßen relativ zu dem Aktivierungsglied 21 entgegen der Vortriebsrichtung V. Das Eingriffselement 12a gleitet längs der Führungskurve 21a. Über diese auf reinem Druckkontakt beruhende Schnittstelle wird die bewegliche Griffkomponente 12 quer zu der Vortriebsrichtung V bewegt, und die Einführeinrichtung 5 schwenkt in die Einführposition. Der Insertionskopf ist am Ende der Ausfahrbewegung, die das Insertionsgehäuse 20 und das Aktivierungsglied 21 relativ zueinander ausführen, aktiviert.

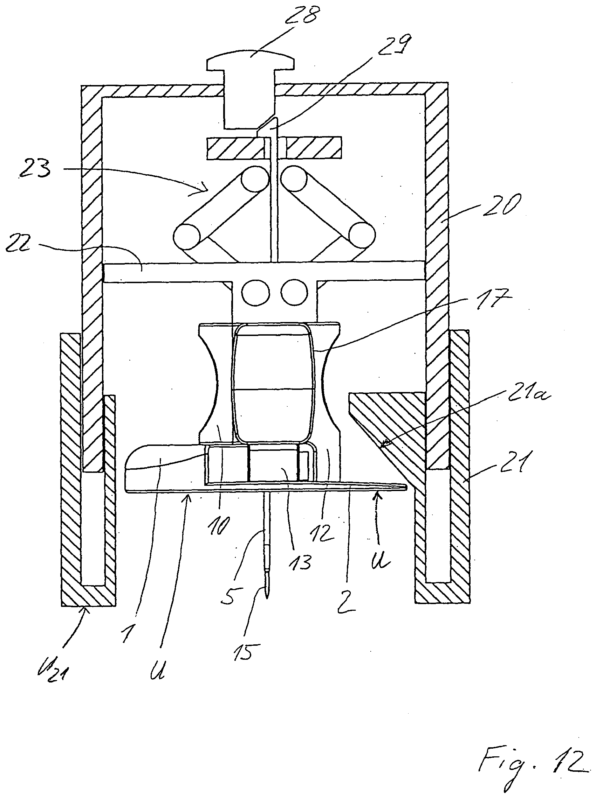

Figur 12 zeigt das System aus Inserter und Insertionskopf in dessen aktiviertem Zustand. Das Insertergehäuse 20 und das Aktivierungsglied 21 nehmen relativ zueinander die ausgefahrene Position ein. In dem ausgefahrenen Zustand umgeben die Wände des Insertergehäuses 20 und des Aktivierungsglieds 21 den aktivierten Insertionskopf bis über das freie Ende der Einführeinrichtung 5 und der Einstecheinrichtung 15 hinaus, d. h. die Spitze der Einstecheinrichtung 15 steht ein kleines Stück weit hinter der Unterseite U₂₁ des Inserters zurück.

Das Insertergehäuse 20 und das Aktivierungsglied 21 sind in der ausgefahrenen Position relativ zueinander blockiert. Relativbewegungen in oder gegen die Vortriebsrichtung V sind in dem blockierten Zustand nicht möglich. Bei Erreichen der ausgefahrenen Position blockieren sich das Insertergehäuse 20 und das Aktivierungsglied 21 aneinander automatisch.

Für die Platzierung des Insertionskopfs setzt der Benutzer den Inserter auf der Hautoberfläche auf. Bei aufgesetztem Inserter drückt der Benutzer auf den Auslöser 28. Der Auslöser 28 wirkt über ein Kurvengelenk, im Ausführungsbeispiel ein einfaches Paar von Schrägen, auf das Blockierglied 29. Unter der Einwirkung des Auslösers 28 bewegt sich das Blockierglied 29 aus dem Blockiereingriff mit dem Insertergehäuse 20, so dass sich das Vortriebselement 22 unter der Einwirkung des Krafterzeugers 23 in die Vortriebsrichtung V bewegen kann. Der Krafterzeuger 23 beschleunigt das Vortriebselement 22 schlagartig. Das Vortriebselement 22 wirkt auf den Insertionskopf wie ein Hammer. Im ersten Abschnitt der Vortriebsbewegung federt die Haltefeder aus dem Halteeingriff mit der Haltestruktur 17 des Insertionskopfs, d. h. der Halteeingriff löst sich. Die Beschleunigung des Vortriebselements 22 in die Vortriebsrichtung V ist so groß, dass der reine Druckkontakt zwischen dem Vortriebselement 22 und dem Insertionskopf sicher erhalten bleibt, bis die Unterseite U des Insertionskopfs auf der gleichen Höhe wie die Unterseite U₂₁ des Inserters und somit auf der Gewebeoberfläche platziert ist. Bereits zuvor durchdringt die Einstecheinrichtung 15 die Hautoberfläche, dringt in das Gewebe ein und nimmt dabei die Einführeinrichtung 5 mit.

Nachdem der Insertionskopf auf der Hautoberfläche platziert ist, greift der Benutzer den Griff 10, 12 und zieht ihn von der Basis 1, 2 ab. Dabei wird die Einstecheinrichtung 15 automatisch aus der Einführeinrichtung 5 heraus- und von der Basis 1, 2 abgezogen.

Um auch das Herausziehen der Einstecheinrichtung 15 zu automatisieren, bleibt der Halteeingriff zwischen der Halteeinrichtung des Inserters und der Haltestruktur 17 des Insertionskopfs in einer vorteilhaften Modifikation des Inserters bestehen und wird nicht, wie im beschriebenen Ausführungsbeispiel, durch die Beschleunigung des Vortriebselements 22 gelöst. In einer derartigen Modifizierung kann die Halteeinrichtung insbesondere ortsfest mit dem Vortriebselement 22 verbunden sein, so dass sie dessen Ausstoßbewegung in die Vortriebsrichtung V mitmacht. Um den Halteeingriff zu lösen, kann der Inserter mit einem Abstreifer ausgestattet sein, der nach dem Wegnehmen des Inserters vom Gewebe bei einem Zusammenschieben von Insertergehäuse 20 und Aktivierungsglied 21 automatisch den Insertionskopf aus dem Halteeingriff löst. Alternativ kann solch ein Abstreifer auch vollkommen unabhängig vom Aktivierungsglied 21 vorgesehen und separat betätigbar sein, um den Halteeingriff zu lösen.

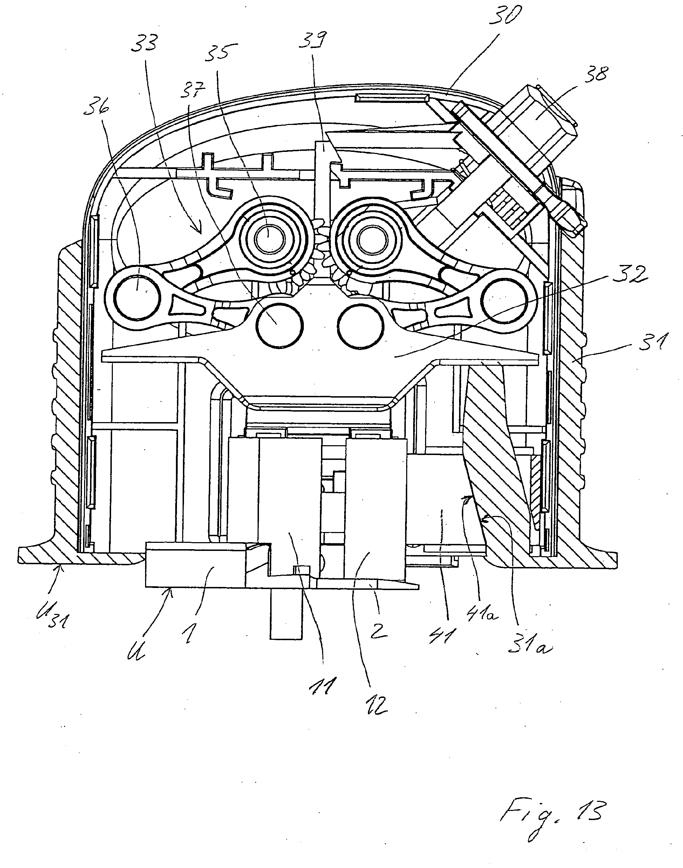

Die Figuren 13 bis 15 zeigen ein aus einem Insertionskopf und einem Inserter bestehendes System eines zweiten Ausführungsbeispiels. Der Insertionskopf ist derjenige der Figuren 7 bis 10, kann aber auch der gleiche wie im ersten Ausführungsbeispiel sein. Nur bei dem Inserter handelt es sich um eine Modifikation. Diejenigen Komponenten des Inserters des zweiten Ausführungsbeispiels, die in Bezug auf ihre Funktion mit den Komponenten des Inserters des ersten Ausführungsbeispiels vergleichbar sind, werden jeweils mit den um die Zahl zehn erhöhten Bezugszeichen des ersten Ausführungsbeispiels belegt. So gelten insbesondere zum Insertergehäuse 30 und dem Aktivierungsglied 31, was deren Form und Verbindung sowie Relativbeweglichkeit anbetrifft, die Ausführungen zum ersten Ausführungsbeispiel. Grundsätzlich das Gleiche gilt auch in Bezug auf das Vortriebselement 32, die Halteeinrichtung und der Krafterzeuger 33 sowie den Auslöser 38 und das Blockierglied 39. Soweit im Folgenden auf Unterschiede nicht hingewiesen wird und sich auch aus den Figuren nichts anderes ergibt, gelten die Ausführungen zum ersten Ausführungsbeispiel gleichermaßen auch für das zweite Ausführungsbeispiel.

Der Inserter des zweiten Ausführungsbeispiels unterscheidet sich von dem Inserter des ersten Ausführungsbeispiels im Wesentlichen im Hinblick auf das Gelenk, über welches das Aktivierungsglied 31 auf den Insertionskopf wirkt, um diesen durch die Aufziehbewegung des Insertergehäuses 30 relativ zu dem Aktivierungsglied 31 zu aktivieren. Im zweiten Ausführungsbeispiel bildet der Inserter selbst das Gelenk, nämlich mit zwei Gelenkelementen 31a und 41a, von denen eines das Aktivierungsglied 31 und das andere ein Effektorglied 41 bildet. Das Effektorglied 41 wird quer zu der Vortriebsrichtung V, im Ausführungsbeispiel rechtwinklig zu der Vortriebsrichtung V, hin und her beweglich von dem Insertionsgehäuse 30 gelagert. Das Gelenk 31a, 41a ist wieder ein Kurvengelenk. Die Führungskurve 31a entspricht der Führungskurve 21a des ersten Ausführungsbeispiels. Das Effektorglied 41 bildet das Eingriffselement 41a, das bei Verlängerung des Inserters an der Führungskurve 31a entlang gleitet und aufgrund des geneigten Verlaufs der Führungskurve 31a bei dem Aufziehen des Inserters eine Querbewegung des Effektorglieds 41 in Richtung auf die zentrale Längsachse des Inserters bewirkt. In dem Gelenk 31a, 41a wird somit die gegen die Vortriebsrichtung V weisende Bewegung, die das Insertergehäuse 30 bei dem Aufziehen relativ zu dem Aktivierungsglied 31 ausführt, in die Querbewegung des Effektorglieds 41 umgewandelt. Dessen Gelenkelement bzw. Eingriffselement 41a ist selbst in der Art einer Führungskurve gebildet, wird hier getriebetechnisch jedoch als Eingriffselement bezeichnet. Das Eingriffselement 41a könnte alternativ auch als beispielsweise einfacher Nocken oder Noppen geformt sein. Ebenso könnte das Eingriffselement 41a als Führungskurve bezeichnet und in einer anderen Modifikation das Gelenkelement 31a als vorragender Nocken oder Noppen geformt werden.

Die Schnittstelle, über welche der Inserter den Insertionskopf aktiviert, ist wieder als reiner Druckkontakt gebildet und besteht zwischen dem Effektorglied 41 und dem Empfangsglied bzw. der beweglichen Griffkomponente 12 des Insertionskopfs. Dieser reine, man könnte auch sagen lose Druckkontakt vereinfacht die Handhabung, da für die Aktivierung keine besondere Gelenkverbindung hergestellt werden muss, es genügt das Aufnehmen des Insertionskopfs in Kombination mit der Betätigung des Aktivierungsglieds 31, was in den Ausführungsbeispielen durch die Aufziehbewegung erfolgt. Der Druckkontakt, d. h. die von dem Effektorglied 41 ausgeübte Druckkraft, wirkt auf die bewegliche Griffkomponente 12 parallel zu der Richtung ihrer Beweglichkeit relativ zu der Basis 1, 2. Durch Zwischenschaltung des Effektorglieds 41 und Verlagerung des Gelenks 31a, 41a gänzlich zum Inserter wird auf die Griffkomponente 12 im zweiten Ausführungsbeispiel vorteilhafterweise quer zu der Richtung der Beweglichkeit der Griffkomponente 12 keine Kraft ausgeübt.

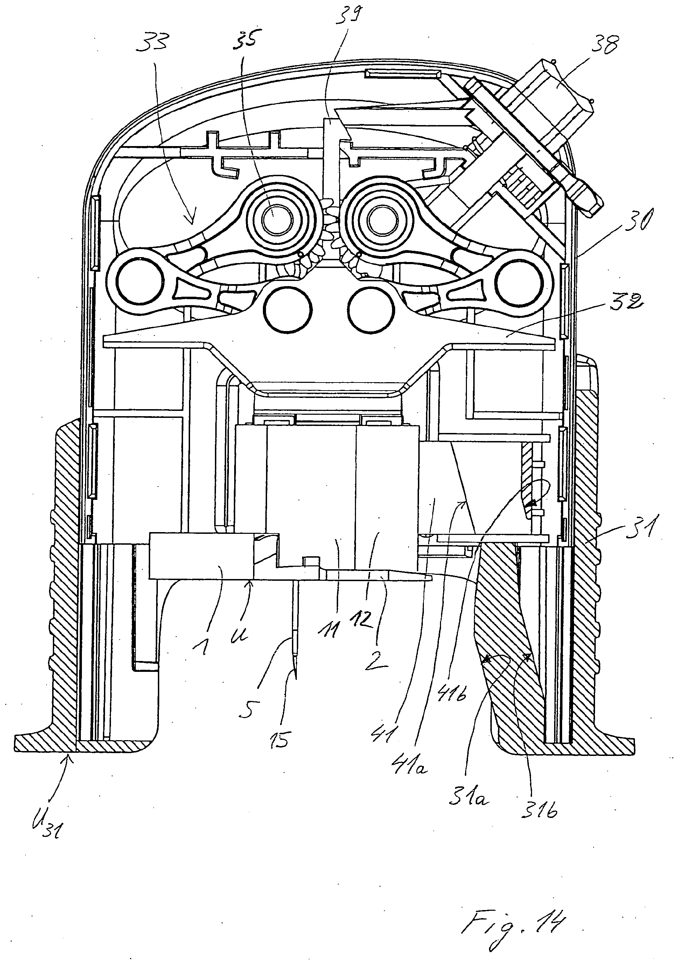

Figur 14 zeigt das System mit aktiviertem Insertionskopf. Im Verlaufe der Aufziehbewegung des Insertergehäuses 30, was auch als Betätigung des Aktivierungsglieds 31 verstanden wird, wurden die Einführeinrichtung 5 und die Einstecheinrichtung 15 in die Einführposition geschwenkt, so dass ihre gemeinsame Längsachse in die Vortriebsrichtung V weist. Die bewegliche Griffkomponente 12 hat wie zum Insertionskopf beschrieben die Verbindung zwischen dem Griff 10, 12 und der Basis 1, 2 gelöst. Der zwischen der Einführeinrichtung 5 und der Einstecheinrichtung 15 bestehende Reibschluss hält jedoch die Basis 1, 2 wie im ersten Ausführungsbeispiel an dem im Halteeingriff befindlichen Griff 10, 12.

Durch Betätigung des Auslösers 38 wird der Blockiereingriff, in dem sich das Blockierglied 39 noch mit dem Insertergehäuse 30 oder einer damit fest verbundenen Struktur befindet, gelöst und der Krafterzeuger 33 beschleunigt das Vortriebselement 32 in die Vortriebsrichtung V. Die Beschleunigung erfolgt wieder schlagartig, so dass auch die Antriebseinrichtung 32, 33 des zweiten Ausführungsbeispiels in der Art eines Hammers wirkt. Die Antriebskraft wird von zwei Schenkelfedern erzeugt, von denen je eine auf eines der beiden Schenkelpaare wirkt. Die im ortsfesten Drehlager 35 befestigten Schenkel 24 sind über einen Zahneingriff miteinander gekoppelt, der für eine synchrone Ausfahrbewegung der beiden Schenkelpaare sorgt.

Um den Inserter nach Platzierung des Insertionskopfs für eine erneute Verwendung bereitmachen zu können, muss das Effektorglied 41 aus der in Figur 14 gezeigten Endposition wieder zurück in die in Figur 13 gezeigte Endposition bewegt werden. Für diese Rückholbewegung bilden das Aktivierungsglied 31 und das Effektorglied 41 ein weiteres Gelenk 31b, 41b, das im Ausführungsbeispiel ebenfalls ein Kurvengelenk ist. Das Aktivierungsglied 31 bildet für das weitere Gelenk die Führungskurve 31b, und das Effektorglied 41 bildet das Eingriffselement 41b. Die Führungskurve 31b verläuft zumindest im Wesentlichen parallel zu der Führungskurve 31a. Die Führungskurven 31a und 31b sind an dem inneren Hülsenteil des Aktivierungsglieds 31 gebildet, die Führungskurve 31a an der Innenfläche und die Führungskurve 31b an der Außenfläche des inneren Hülsenteils. Sie liegen sich in etwa auf der gleichen Höhe, bezogen auf die Vortriebsrichtung V, gegenüber. Das Eingriffselement 41b liegt dem Eingriffselement 41a ebenfalls gegenüber mit einem lichten Abstand, so dass das innere Hülsenteil des Aktivierungsglieds 31 zwischen den beiden Eingriffselementen 41a und 41b ein- und ausfahren kann.

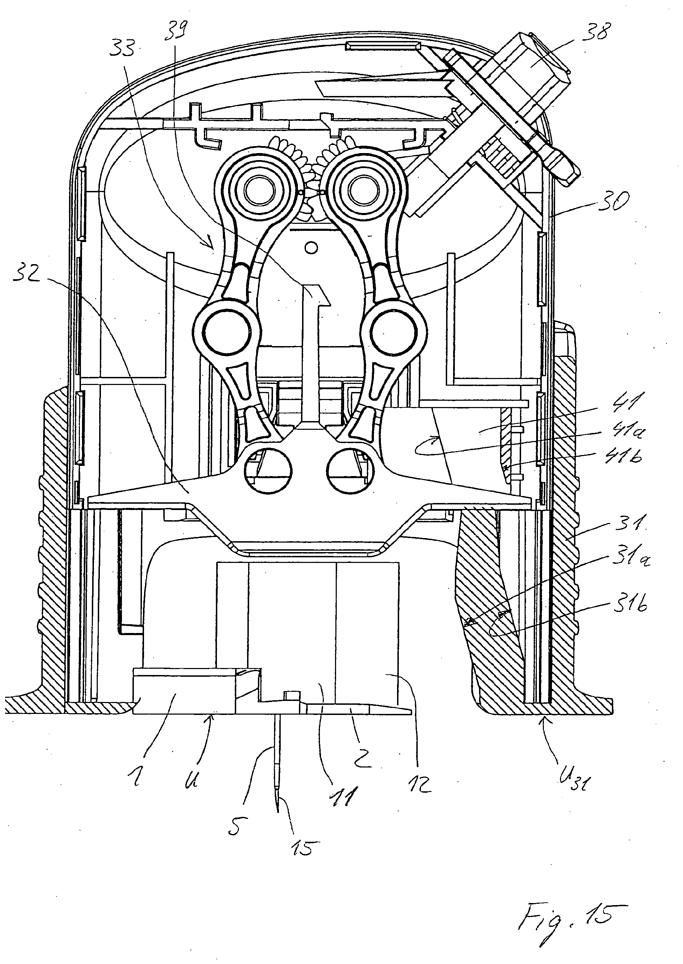

Figur 15 zeigt das System des zweiten Ausführungsbeispiels mit dem platzierten Insertionskopf. Der Inserter wird von dem Insertionskopf entfernt. Anschließend zieht der Benutzer den Griff 10, 12 von der Basis 1, 2 ab und schließt den Insertionskopf an einen Katheter einer Infusionspumpe an. In einer auch bereits zum ersten Ausführungsbeispiel erwähnten Modifikation, in welcher die Halteeinrichtung ortsfest mit dem Vortriebselement 32 verbunden ist und demgemäß den Griff 10, 12 noch halten kann, werden der Inserter und damit gemeinsam der noch gehaltene Griff 10, 12 von der Basis entfernt. Anschließend wird vorzugsweise mittels eines zusätzlichen Abstreifers der Halteeingriff gelöst und der Griff 10, 12 mit der Einstecheinrichtung 15 oder nur dieser alleine entsorgt.

Um den Inserter für die Verwendung mit einem weiteren Insertionskopf vorzubereiten, schiebt der Benutzer das Insertergehäuse 30 und das Aktivierungsglied 31 wieder zusammen in die eingefahrene Position, wie sie mit eingesetztem Insertionskopf in Figur 13 dargestellt ist. Bei der Einfahrbewegung fährt das innere Hülsenteil des Aktivierungsglieds 31 zwischen die Eingriffselemente 41a und 41b des Effektorglieds 41. Bei dieser Einfahrbewegung entsteht die weitere Gelenkverbindung zwischen der Führungskurve 31b und dem Eingriffselement 41b. Bei der Einfahrbewegung wird somit in dem Gelenk 31b, 41b das Effektorglied 41 wieder in die in Figur 13 eingenommene Endposition zurückbewegt, d. h. in Bezug auf die zentrale Längsachse des Inserters quer, vorzugsweise radial, nach auswärts bewegt.

Das unter der Einwirkung der Federeinrichtung 33 in die Vortriebsrichtung V ausgefahrene Vortriebselement 32 liegt einer Stirnseite des inneren Hülsenteils, die von der Unterseite U₃₁ des Aktivierungsglieds 31 abgewandt ist, gegenüber. Durch Anschlagkontakt gegen diese Stirnseite wird die Vortriebsbewegung des Vortriebselements 32 gestoppt. Das Aktivierungsglied 31 ist geometrisch so bemessen, dass es in der ausgefahrenen Position des Teleskops 30, 31 das Vortriebselement 32 genau dann stoppt, wenn die Unterseite U des Insertionskopfs die Höhe der Unterseite U₃₁ erreicht hat und daher bei aufgesetztem Inserter die Hautoberfläche gerade kontaktiert. Bei der Einfahrbewegung des Insertergehäuses 30 relativ zum Aktivierungsglied 31 bzw. des Aktivierungsglieds 31 relativ zu dem Insertergehäuse 30 wird das Vortriebselement 32 wegen des Anschlagkontakts vom Aktivierungsglied 31 gegen die Kraft des Krafterzeugers 33 tiefer in das Insertergehäuse 30 gedrückt, bis das Blockierglied 39 wieder im Blockiereingriff ist, wie ihn beispielhaft die Figuren 13 und 14 zeigen.

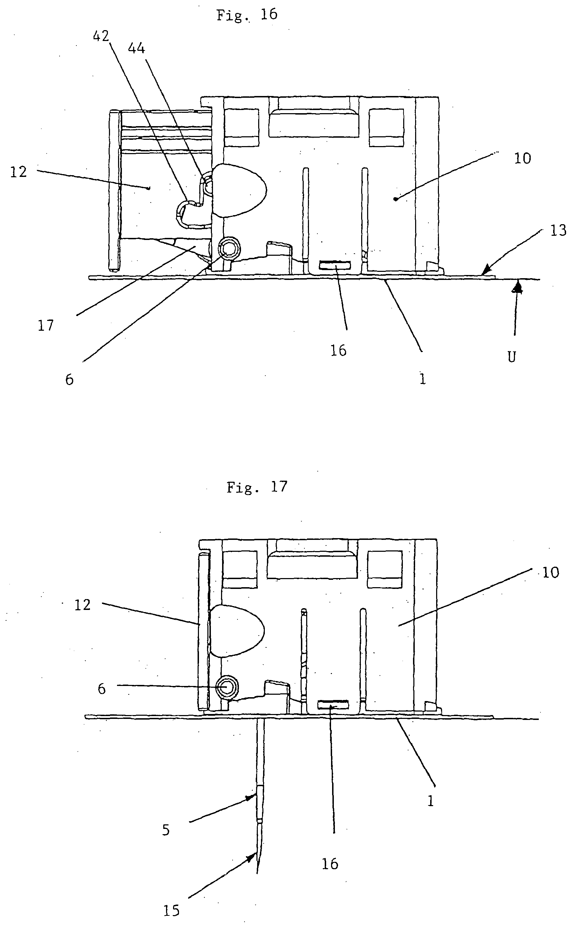

In Fig. 16 ist eine weitere Ausführungsform des Insertionskopfes nach der Erfindung dargestellt. Der Insertionskopf umfasst eine bewegliche Griffkomponente 12, die in eine weitere Griffkomponente 10 eingeschoben werden kann. Durch das Einschieben der beweglichen Griffkomponente 12 in die weitere Griffkomponente 10 wird ein Kanülengehäuse 17b um ein Gelenk 6 verschwenkt. Das Kanülengehäuse 17b trägt dabei noch in der Schutzposition die Einführeinrichtung 5 und die Einstecheinrichtung 15. Während der Betätigung der beweglichen Griffkomponente 12 wird der Kulissenstein 44 in der Kulissenführung 42 im Wesentlichen nach unten in Richtung des Gelenkelementes 6 verfahren, was zu einer Dreh- bzw. Schwenkbewegung für die Haltestruktur 17 und damit für die Einführeinrichtung und die Einstechnadel führt. Sobald der Kulissenstein bzw. der Zapfen 44 im Kurvenbereich der Kulissenführung 42 angelangt ist, ist die Schwenkbewegung von der Schutzposition in die Einführposition vollendet. Die weitere Griffkomponente 10 ist noch mit einer Verbindungseinrichtung 16b ausgestattet, das im Eingriff mit einem korrespondierenden Abschnitt 49 an der Basis 1 steht, um somit die Basis reversibel mit dem Griff 10, 12 zu verbinden.

Natürlich kann der Kulissenstein auch eine andere Position aufweisen.

So könnte der Kulissenstein 44 auch weiter unten angeordnet sein und beim Verschwenken der Bestandteile 17a, 17b dann im Wesentlichen nach oben wandern. Hier sind verschiedene Konfigurationen für die Kulissenführung und den Kulissenstein möglich.

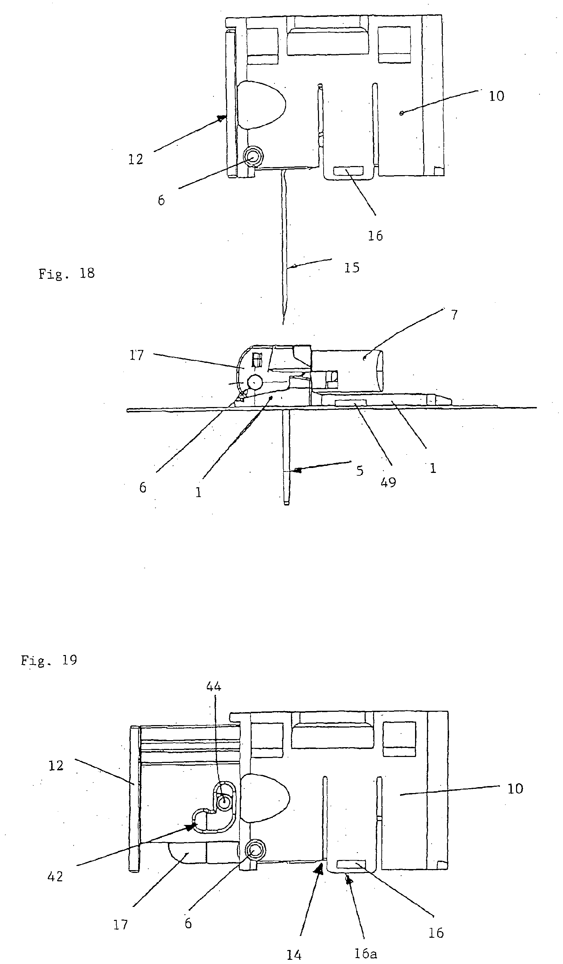

Gemäß Fig. 17 ist die bewegliche Griffkomponente 12 in die weitere Griffkomponente 10 versenkt, wobei die Einführbewegung der bewegliche Griffkomponente 12 in die weitere Griffkomponente 10 zu dem Herausschwenken der Einführeinrichtung 5 zusammen mit der Einstecheinrichtung 15 aus der Aufnahme 14 (siehe Fig. 19) in die Einführposition geführt hat. Damit stellt diese Ausführungsform gemäß den Fig. 16 ff. eine zu den voranstehend erörterten Ausführungsformen alternative Kopplung zwischen den Griffkomponenten und der verschwenkbaren Einführeinrichtung zur Verfügung.

Gemäß Fig. 18 ist dargestellt, wie der Griff, bestehend aus den Griffkomponente 12 und 10 von der Basis 1 entkoppelt werden kann. Die Basis 1 enthält eine Aussparung 49, mit der eine Verbindungseinrichtung 16b an dem Griff 10, 12 im Eingriff war (siehe Fig. 16 und 17), während dieser Eingriff in der Darstellung gemäß Fig. 18 aufgehoben worden ist, so dass der Griff 10, 12 von der Basis 1 abnehmbar ist.

Die Fig. 19 zeigt den Griff 10, 12 gemäß Fig. 18, wobei hier die bewegliche Griffkomponente 12 aus der weiteren Griffkomponenten 10 herausgezogen worden ist, so dass der Kulissenstein bzw. der Zapfen 44 in der Kulissenführung 42 im Wesentlichen nach oben geführt worden ist, wodurch das Kanülengehäuse 17b und der Nadelhalter 17a mit der daran festgelegten Einstecheinrichtung 15 wieder verschwenkt worden sind, und zwar in die ursprüngliche Schutzposition in der Aufnahme 14 oder eine dieser nahen Sperrposition. Die Verbindungseinrichtung 16b, die am Ende eines Federstegs 16a sitzt, ist nun ohne Funktion, da sie keine Verbindung mehr zu der Basis 1 halten muss.

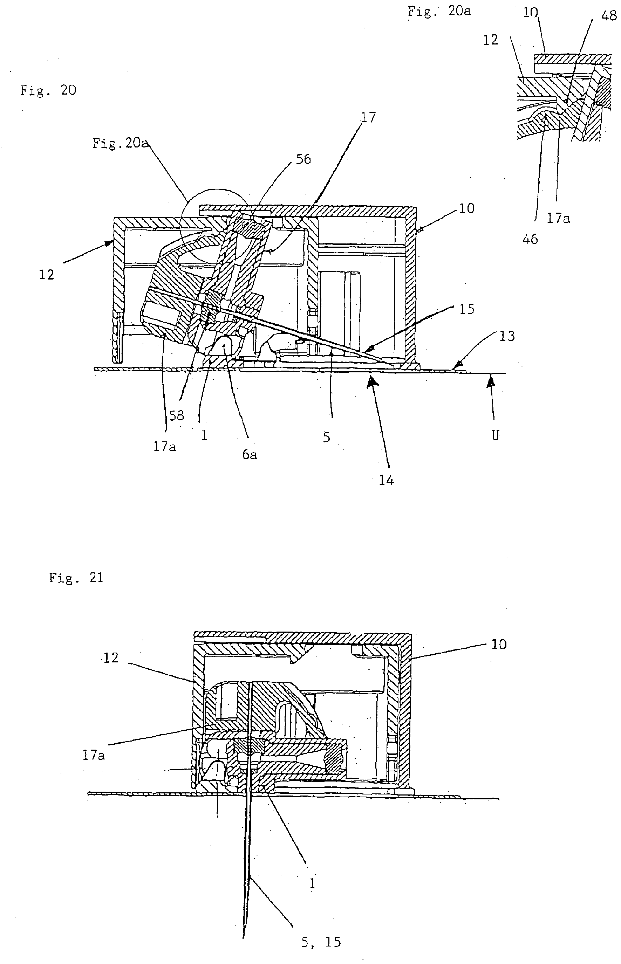

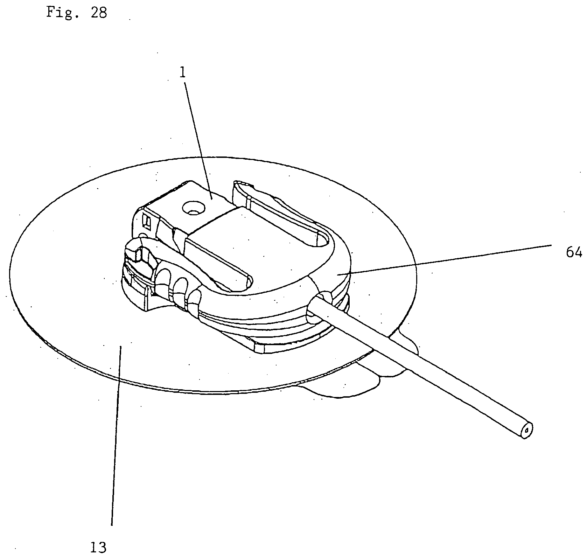

Gemäß Fig. 20 ist der Insertionskopf gemäß einer weiteren Ausführungsform nach der Erfindung in einer Situation vor einer Applikation bzw. während der Lagerung dargestellt. Die Einführeinrichtung 5 mit der darin teilweisen aufgenommenen Einstecheinrichtung 15 befindet sich in der Schutzposition in der Aufnahme 14. Auf der Lasche 13 bzw. dem Pflaster 13 ist eine Schutzfolie auf der Unterseite U aufgebracht, um die Klebestellen des Pflasters 13 aktiv zu halten. Gemäß Fig. 20 ist ferner ein Septum 58 vorgesehen, dass das Einführen und Herausnehmen der Einstecheinrichtung 15 ermöglichen soll, wobei anschließend eine Abdichtung für die Einführeinrichtung 5 gewährleistet sein soll. Entsprechend ist auch für den senkrecht an die Einstecheinrichtung 15 anschließenden Anschlussabschnitt ein Septum 56 vorgesehen, das einen dichten Anschluss an eine Zufuhrleitung bzw. einen Konvektor (siehe Fig. 28) ermöglichen soll.

An einem auf dem Kanülengehäuse 17b angeordneten Nadelhalter 17a, der die Einstecheinrichtung 15 hält, ist auf der der Basis 1 abgewandten Seite eine Sicherungsstruktur 46 vorgesehen, die sich aus dem Bildausschnitt gemäß Fig. 20a ergibt. Ein Eingriffselement 48 ist im dargestellten Zustand im Eingriff mit der Sicherungsstruktur 46 und ergibt einen Widerstand, der einem zufälligen Einführen der bewegliche Griffkomponente 12 in die weitere Griffkomponente 10 entgegenwirkt. Der Bediener des erfindungsgemäßen Insertionskopfes gemäß dieser Ausführungsform muss anfangs einen erhöhten Kraftaufwand bereitstellen, um den Eingriff zwischen dem Eingriffselement 48 und der Sicherungsstruktur 46 zu überwinden und um damit den Schwenkvorgang für die Einführeinrichtung 5 nebst Einstecheinrichtung 15 aus der Schutzposition in der Aufnahme 14, wie sie in Fig. 20 dargestellt ist, in die Applikationsposition, wie sie in Fig. 21 dargestellt ist, durchzuführen.

Auch ist es möglich, die Sicherungsstruktur 46 mit den Elementen 52, 54 entsprechenden Rasteinrichtungen zu realisieren, die beispielsweise an der weiteren und der bewegliche Griffkomponente 10, 12 vorgesehen sein können und durch das Einschieben der einen in die andere entriegelt werden können.

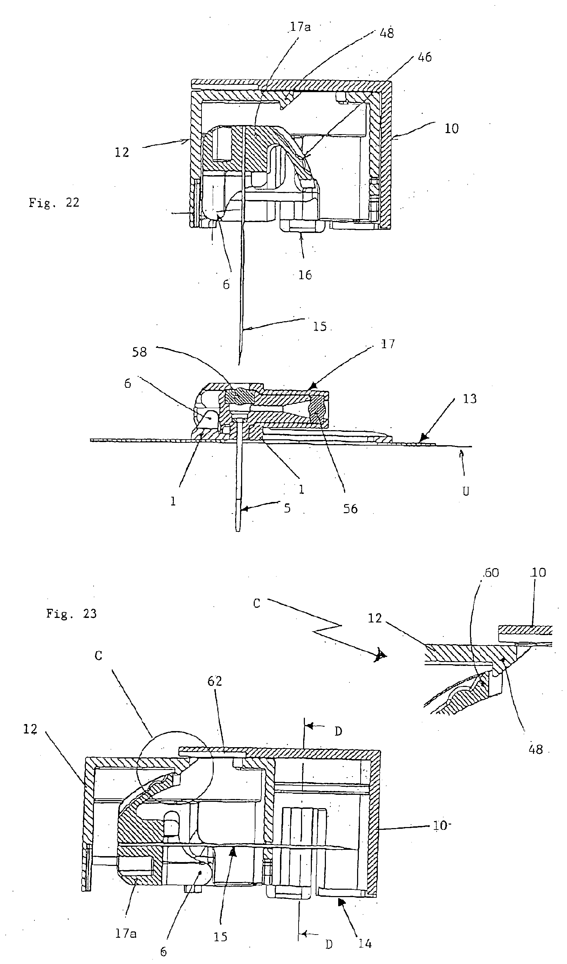

Die in Fig. 21 in das Gewebe eines Patienten eingestochene Einführeinrichtung nebst Einstecheinrichtung 5, 15 ist gemäß Fig. 22 demontiert. D.h., die Einstecheinrichtung bzw. Einstechnadel 15 ist aus der Einführeinrichtung 5 herausgezogen. Das Septum 58 schließt sich und die Einführeinrichtung 5 ist bereit, ein Medikament in den Körper eines Patienten einzuführen.

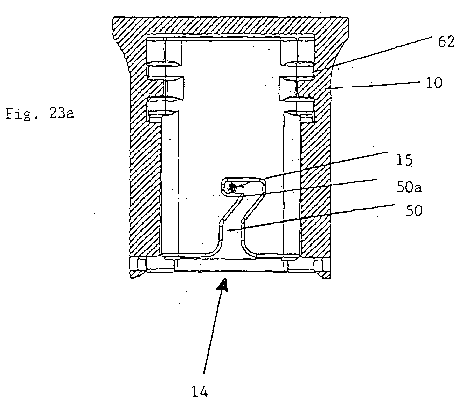

In Fig. 23 ist erkennbar, wie die bewegliche Griffkomponente 12 wieder aus der weiteren Griffkomponente 10 herausgezogen worden ist, so dass das Kanülengehäuse 17b wieder in seine der Schutzposition zugeordnete Stellung zurückgeschwenkt worden ist. Die Einstecheinrichtung 15 über die ursprüngliche Schutzposition in eine Position oberhalb der Schutzposition, d.h., oberhalb einer nachfolgend erläuterten Rastschulter 60. In dieser Position wird die Einstecheinrichtung 15, wie in dem Vergrößerungsbild C erkennbar, dadurch gesichert, dass eine Rastschulter 60 am Ende des Nadelhalters 17a in Eingriff mit dem Eingriffselement 48 gelangt ist. In dieser Position ist es nun nicht mehr möglich, die kontaminierte Einstecheinrichtung 15 wieder aus dem Griff 10, 12 auszulenken, weil eine Sperrwirkung durch das Eingriffselement 48 in Verbindung mit der Rastschulter 60 bereitgestellt wird. Die Einstecheinrichtung 15 wird hinter der Rastschulter in einer Sperrposition gehalten. Gemäß Fig. 23a wird ein Schnitt D-D gemäß Fig. 23 wiedergegeben, um eine Sicherungskulisse 50 darzustellen, die dazu dient, die benutzte bzw. kontaminierte Einstecheinrichtung 15 in einen gesicherten Kulissenabschnitt hinter einer Sicherungsschulter 50a zu überführen. Demgegenüber kann die ungenutzte, sterile Einstecheinrichtung vor der Benutzung in einer anderen Position vor der Sicherungsschulter 50a gehalten werden.

In der Schnittdarstellung gemäß Fig. 23a sind auch Führungen 62 zu erkennen, die eine Führung der bewegliche Griffkomponente innerhalb der weiteren Griffkomponente 10 ermöglichen. Wird die Einstecheinrichtung 15 in eine inaktive Position in der Aufnahme 14 zurückgeschwenkt, wird sie sich, sich flexibel verformend, durch die Sicherungskulisse 50 bewegen, um anschließend im oberen Bereich der Sicherungskulisse 50 hinter der Sicherungsschulter 50a entlastet zu werden und in eine unmittelbar in Fig. 23a dargestellte Position zurückschnellen, um dann sicher in der dargestellten Position zu verharren und dort blockiert zu sein.

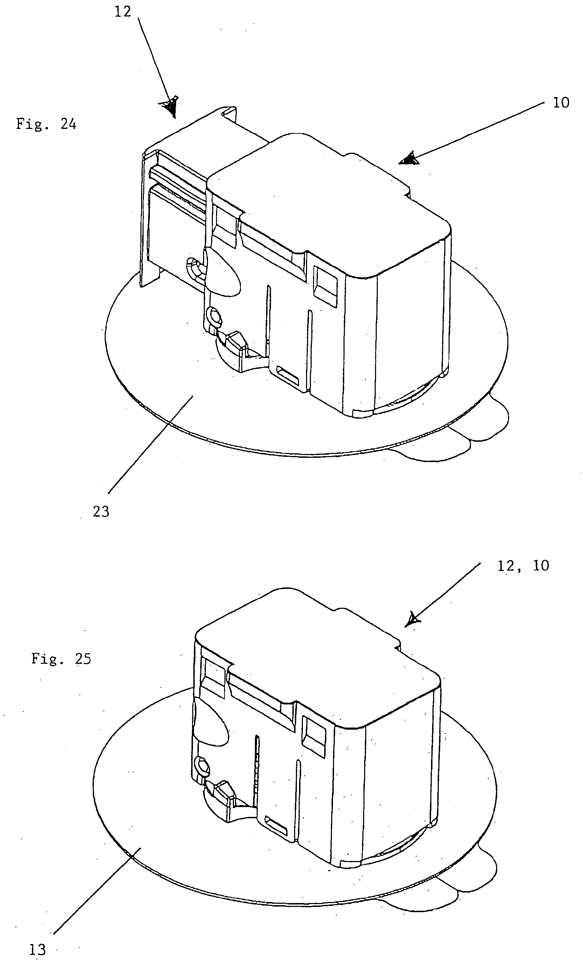

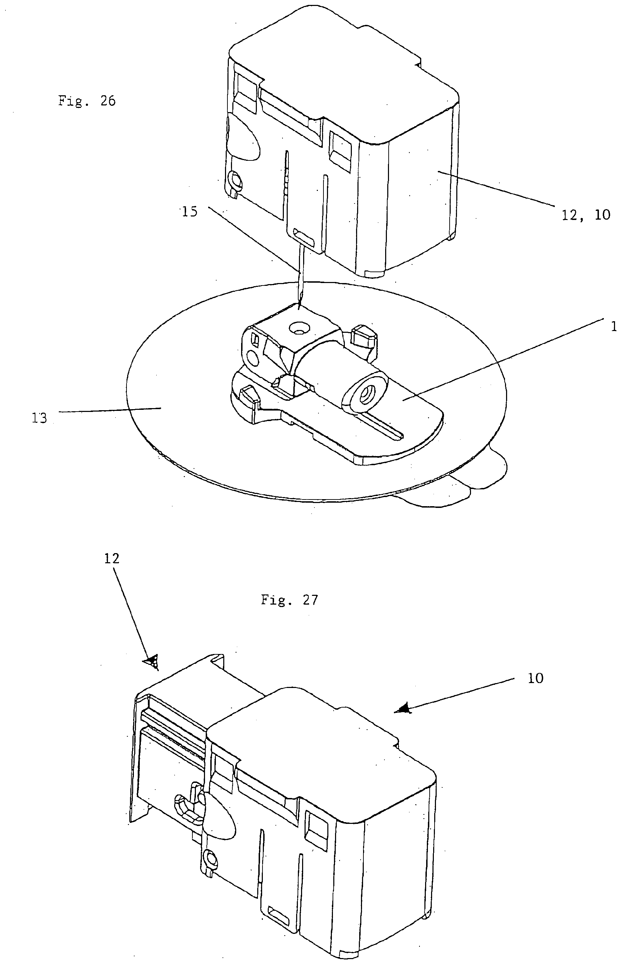

Die Fig. 24 bis 28 zeigen verschiedene Stadien der Applikation eines erfindungsgemäßen Insertionskopfes. Gemäß Fig. 24 ist die bewegliche Griffkomponente 12 in einer aus der weiteren Griffkomponente 10 herausragenden Position dargestellt, in der die Einführeinrichtung 5 nebst Einstechnadel 15 in ihrer Schutzposition sind. Bei der Darstellung gemäß Fig. 25 ist die bewegliche Griffkomponente 12 in die weitere Griffkomponente 10 hinein bewegt worden, so dass, wie sich aus Fig. 26 ergibt, die Einführeinrichtung nebst Einstecheinrichtung in den Körper eines Patienten eingestochen werden können. Die Fig. 26 zeigt nun den Zustand, nach dem die Einführeinrichtung nebst Einstecheinrichtung 15 in dem Körper eines Patienten eingestochen worden sind, wobei nun die Einstecheinrichtung 15 ohne weitere Funktion ist und aus der Einführeinrichtung herausgezogen werden kann, d.h., die Basis 1 und der Griff 10, 12 können voneinander getrennt werden.

Gemäß Fig. 27 ist die bewegliche Griffkomponente 12 nun aus der weiteren Griffkomponente 10 herausgezogen worden, wobei die Einstecheinrichtung 15 wieder in eine Schutzposition überführt worden ist.

Gemäß Fig. 28 kann nun ein Konnektor 64 mit einer Zuführleitung an die Basis angedockt werden, um ein Medikament, beispielsweise Insulin, zuzuführen.

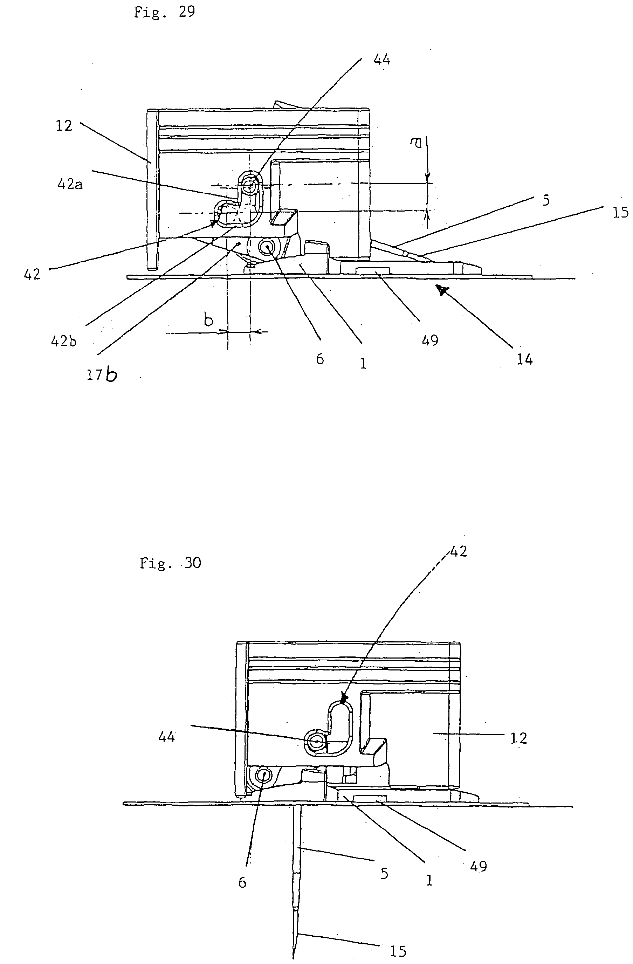

In der Fig. 29 ist dargestellt, dass die Führungskulisse 42 einen ersten Abschnitt 42a der Länge a hat. Diese Strecke a dient dazu, das Kanülengehäuse 17b und damit die Einführeinrichtung 5 nebst der Einstecheinrichtung 15 durch Betätigen bzw. Vorschub der bewegliche Griffkomponente 12 im dargestellten Falle nach rechts zu verschwenken, so dass der Kulissenstein 44 sich im Wesentlichen nach unten in Richtung des Gelenkelementes 6 bewegt, während die Einführeinrichtung 5 aus der Schutzposition in die Applikationsposition bzw. Einführposition überführt wird. Ein zweiter Abschnitt 42b der Kulissenführung 42 steht zur Verfügung, um über die zweite Strecke b einen Spielraum zu verschaffen, um eine Entriegelung vornehmen zu können, um die Verbindungseinrichtung 16b, aus der Eingriffsausnehmung 49 herauslenken zu können.

Dementsprechend ist gemäß Fig. 30, in der der Kulissenstein 44 am anderen Ende der Kulissenführung 42 relativ zu der Darstellung gemäß Fig. 29 angelangt ist, die Einführeinrichtung 5 nebst Einstecheinrichtung 15 nicht nur in ihrer Applikationsposition, sondern der Griff 10, 12 ist zudem gegenüber der Basis 1 bereits entriegelt und kann abgenommen werden, wobei die Einstecheinrichtung 15 aus der Einführeinrichtung 5 herausgezogen wird.

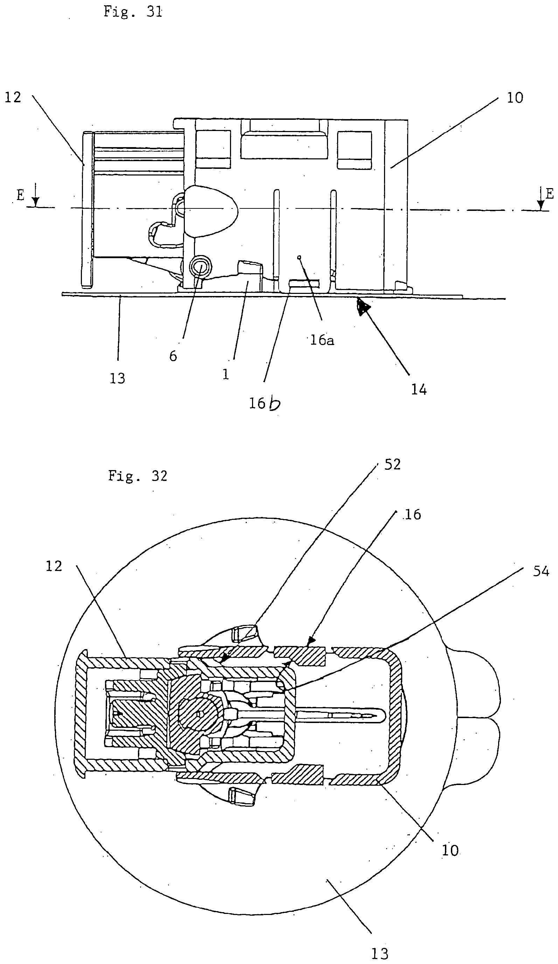

Bei der Ausführungsform gemäß Fig. 31 ist zusätzlich ein Entriegelungsmechanismus dargestellt, der sich insbesondere im Schnitt E-E ergibt, und der in Fig. 32 wiedergegeben ist. Wie zu erkennen ist, weist die bewegliche Griffkomponente 12 eine Steuerrampe auf, die nach dem Einführen der bewegliche Griffkomponente 12 in die weitere Griffkomponente 10 gegen eine Auslenkrampe 54 anläuft, um auf diese Weise das Verbindungselement 16 aus seinem Eingriff mit der Eingriffsausnehmung 49 an der Basis 1 herauszubefördern. Während die Bewegung zum Verschwenken der Einführeinrichtung 5 nebst Einstecheinrichtung 15 der Strecke 42a mit der Länge a entspricht, entspricht die Bewegung zum Führen der Steuerrampe 52 gegen die Auslenkrampe 54 dem Abschnitt 42b der Führungskulisse 42 gemäß Fig. 29.

### Bezugszeichen:

- 1: Basis, Aufnahme
- 2: Basis, Flachteil
- 3: Ausnehmung
- 4: Ausnehmung
- 5: Einführeinrichtung
- 6: Gelenkelement
- 6a: Gelenkelementgegenstück
- 7: Zuführung
- 8: Zahnrad
- 9: Verbindungselement
- 10: weitere Griffkomponente
- 11: weitere Griffkomponente
- 12: bewegliche Griffkomponente, Empfangsglied
- 12a: Gelenkelement, Eingriffselement, Druckkontaktfläche
- 13: Lasche
- 14: Aufnahme
- 15: Einstecheinrichtung
- 16: Verbindungselement
- 16a: Federsteg
- 16b: Verbindungseinrichtung
- 17: Haltestruktur
- 17a: Nadelhalter
- 17b: Kanülengehäuse
- 18: Zahnstange
- 19: Verbindungselement
- 20: Insertergehäuse
- 21: Aktivierungsglied
- 21a: Gelenkelement, Führungskurve
- 22: Vortriebselement
- 23: Krafterzeuger
- 24: Schenkel
- 25: Drehgelenk
- 26: Drehgelenk
- 27: Drehgelenk
- 28: Auslöser
- 29: Blockierglied
- 30: Insertergehäuse
- 31: Aktivierungsglied
- 31a: Gelenkelement, Führungskurve
- 31b: Gelenkelement, Führungskurve
- 32: Vortriebselement
- 33: Krafterzeuger
- 34: Schenkel
- 35: Drehgelenk
- 36: Drehgelenk
- 37: Drehgelenk
- 38: Auslöser
- 39: Blockierglied
- 40: -
- 41: Effektorglied
- 41a: Gelenkelement, Eingriffselement
- 41b: Gelenkelement, Eingriffselement
- 42: Kulissenführung
- 42a: Schwenkführungsabschnitt
- 42b: Entrastungsführungsabschnitt
- 44: Kulissenstein, Zapfen
- 46: Sicherungsstruktur
- 48: Eingriffselement
- 49: Eingriffsausnehmung
- 50: Sicherungskulisse
- 50a: Sicherungsschulter
- 52: Steuerrampe
- 54: Aulenkrampe
- 56, 58: Septum
- 60: Rastschulter
- 62: Führung
- 64: Konnektor

- U: Unterseite
- V: Vortriebsrichtung

## Patentansprüche

1. Insertionskopf für medizinische oder pharmazeutische Anwendungen, der Insertionskopf umfassend:
a) eine Basis (1, 2) mit einer auf organischem Gewebe platzierbaren Unterseite (U)
b) eine in das Gewebe einführbare Einführeinrichtung (5), wobei die Basis (1,2) die Einführeinrichtung (5) permanent und bewegbar lagert und wobei die Basis (1, 2) und ein Gelenkelement (6) miteinander ein Gelenk bilden und die Einführeinrichtung (5) von dem Gelenkelement (6) abragt, vorzugsweise indem eine Längsrichtung der Einführeinrichtung (5) eine Bewegungsachse des Gelenks schneidet oder kreuzt,
c) eine zu der Unterseite (U) der Basis (1,2) offene und von der Basis (1, 2) lösbare Aufnahme (14), die zumindest ein freies Ende der Einführeinrichtung (5) in einer Schutzposition aufnimmt,
d) einen Griff (11, 12, 14), der mit der Basis (1,2) und der von der Basis (1,2) gelagerten Einführeinrichtung (5) lösbar verbunden ist und die lösbare Aufnahme (14) bildet;
wobei
e) die Einführeinrichtung (5) relativ zu der Basis (1,2) aus ihrer Schutzposition, in der ein freies Ende der Einführeinrichung (5) hinter der Unterseite (U) der Basis (1,2) zurücksteht, in eine Einführposition schwenkbar ist, in der das freie Ende der Einführeinrichtung (5) über die Unterseite (U) der Basis (1, 2) vorragt.

2. Insertionskopf nach Anspruch 1, **dadurch gekennzeichnet, dass** die Basis (1, 2) eine zu der Unterseite (U) der Basis (1,2) offene weitere Aufnahme (1) bildet, die einen Abschnitt der Einführeinrichtung (5) in deren Einführposition aufnimmt und dass die Einführeinrichtung (5) durch eine Ausnehmung (4) der weiteren Aufnahme (1) in die lösbare Aufnahme (14) ragt.

3. Insertionskopf nach dem vorhergehenden Anspruch, **dadurch gekennzeichnet, dass** die lösbare Aufnahme (14) die weitere Aufnahme (1) in Längsrichtung der Einführeinrichtung (5) verlängert, vorzugsweise die weitere Aufnahme (1) aufnimmt.

4. Insertionskopf nach einem der zwei vorhergehenden Ansprüche, **dadurch gekennzeichnet, dass** die weitere Aufnahme (1) eine äußere Oberflache aufweist, die von ihrer Oberseite in Richtung auf die Unterseite (U) der Basis (1,2) nach außen rund vorgewölbt abfallt.

5. Insertionskopf nach einem der vorhergehenden Ansprüche, **dadurch gekennzeichnet, dass** der Griff (11, 12, 14) einen von einer Oberseite der Basis (1, 2) aufragenden Griffteil (11, 12) aufweist und die lösbare Aufnahme (14) von dem Griffteil (11, 12) seitlich abragt.

6. Insertionskopf nach einem der vorhergehenden Ansprüche, **dadurch gekennzeichnet, dass** der Griff (11, 12, 14) eine relativ zu der Basis (1, 2) bewegliche Griffkomponente (12) umfasst oder als solche gebildet ist und die bewegliche Griffkomponente (12) mit der Einführeinrichtung (5) mittels eines Getriebes (8, 18) so gekoppelt ist, dass eine relativ zu der Basis (1, 2) stattfindende Bewegung der beweglichen Griffkomponente (12) die Bewegung der Einführeinrichtung (5) bewirkt.

7. Insertionskopf nach dem vorhergehenden Anspruch, **dadurch gekennzeichnet, dass** das Getriebe (8, 18) die bewegliche Griffkomponente (12) mittels Zahneingriffs, vorzugsweise eines Zahnrads (8) und einer Zahnstange (18), mit der Einführeinrichtung (5) koppelt.

8. Insertionskopf nach einem der zwei vorhergehenden Ansprüche, **dadurch gekennzeichnet, dass** die bewegliche Griffkomponente (12) mit einer Zahnstange (18), die vorzugsweise an der beweglichen Griffkomponente (12) geformt ist, und die Einführeinrichtung (5) mit einem mit der Zahnstange (18) in dem Zahneingriff befindlichen Zahnrad (8) verbunden ist.

9. Insertionskopf nach einem der drei vorhergehenden Ansprüche, **dadurch gekennzeichnet, dass** die Einführeinrichtung (5) um eine Rotationsachse schwenkbar und drehsteif mit einem um die Rotationsachse drehbaren, in dem Zahneingriff befindlichen Zahnrad (8) verbunden ist.

10. Insertionskopf nach einem der vorhergehenden Ansprüche, **dadurch gekennzeichnet, dass** der Griff (11, 12, 14) eine relativ zu der Basis (1, 2) bewegliche Griffkomponente (12) aufweist und eine zwischen der Basis (1, 2) und dem Griff (11, 12) bestehende Verbindung sich bei einer Bewegung der beweglichen Griffkomponente (12) löst.

11. Insertionskopf nach dem vorhergehenden Anspruch, **dadurch gekennzeichnet, dass** an der Basis (1, 2) ein erstes Verbindungselement (9) und an dem Griff (11, 12, 14) ein zweites Verbindungselement (19) geformt ist, dass die Verbindungselemente (9, 19) miteinander in einem die Verbindung schaffenden Eingriff sind und dass wenigstens eines der Verbindungselemente (9, 19) vorzugsweise gegen eine Elastizitätskraft aus dem Eingriff bewegbar ist.

12. Insertionskopf nach einem der zwei vorhergehenden Ansprüche, **dadurch gekennzeichnet, dass** ein erstes Verbindungselement (9) und ein zweites Verbindungselement (19) miteinander in einem die Verbindung schaffenden Eingriff sind und die bewegliche Griffkomponente (12) bei ihrer Bewegung eines der Verbindungselemente (9, 19) kontaktiert und vorzugsweise gegen eine Elastizitätskraft aus dem Eingriff bewegt.

13. Insertionskopf nach einem der Ansprüche 7 bis 13, **dadurch gekennzeichnet, dass** der Griff (11, 12, 14) eine weitere Griffkomponente (11, 14) umfasst und die bewegliche Griffkomponente (12) und die weitere Griffkomponente (11, 14) zwischen zwei Fingern einer Hand greifbar sind, wobei mit je einem der Finger gegen eine der Griffkomponenten (11, 12, 14) drückend die bewegliche Griffkomponente (12) gegen die weitere Griffkomponente (11, 14) bewegbar ist.

14. Insertionskopf nach einem der Ansprüche 6 bis 13, **dadurch gekennzeichnet, dass** die bewegliche Griffkomponente (12) relativ zu der Basis (1,2) verschiebbar ist.

15. Insertionskopf nach einem der Ansprüche 6 bis 14, **dadurch gekennzeichnet, dass** die bewegliche Griffkomponente (12) linear geführt ist.

16. Insertionskopf nach einem der Ansprüche 6 bis 15, **dadurch gekennzeichnet, dass** die bewegliche Griffkomponente (12) zumindest im Wesentlichen parallel zu der Unterseite (U) der Basis (1,2) beweglich ist.

17. Insertionskopf nach einem der Ansprüche 6 bis 16, **dadurch gekennzeichnet, dass** der Griff (11, 12, 14) eine weitere Griffkomponente (11, 14) umfasst, die relativ zu der Basis (1, 2) im verbundenen Zustand unbeweglich ist und die lösbare Aufnahme (14) bildet.

18. Insertionskopf nach einem der vorhergehenden Ansprüche, **dadurch gekennzeichnet, dass** der Griff (11, 12, 14) von einer Oberseite der Basis (1,2) aufragt.

19. Insertionskopf nach einem der vorhergehenden Ansprüche, **dadurch gekennzeichnet, dass** die Einführeinrichtung (5) flexibel ist und von einer Einstecheinrichtung (15) stabilisiert wird, die gemeinsam mit der Einführeinrichtung (5) aus der Schutzposition in die Einführposition bewegbar ist.

20. Insertionskopf nach dem vorhergehenden Anspruch, **dadurch gekennzeichnet, dass** die Einstecheinrichtung (15) sich bei der Bewegung der Einführeinrichtung (5) in die Einführposition mit der lösbaren Aufnahme (14) verbindet.

21. Insertionskopf nach einem der zwei vorhergehen den Ansprüche, **dadurch gekennzeichnet, dass** die Einstecheinrichtung (15) mit der lösbaren Aufnahme (14) von der Basis (1,2) und der Einführeinrichtung (5) lösbar ist.

22. Insertionskopf nach einem der vorhergehenden Ansprüche, **dadurch gekennzeichnet, dass** die Einführeinrichtung (5) in eine Längsrichtung langgestreckt ist und die Längsrichtung mit der Unterseite (U) der Basis (1, 2) einen spitzen Winkel von weniger als 50' einschließt, wenn das freie Ende der Einführeinrichtung (5) bei der Bewegung in die Einführposition über die Unterseite (U) vortritt.

23. Insertionskopf nach einem der vorhergehenden Ansprüche, **dadurch gekennzeichnet, dass** die Basis (1,2) die Einführeinrichtung (5) um eine Rotationsachse schwenkbar lagert und die Einführeinrichtung (5) eine Langsachse aufweist, welche die Rotationsachse schneidet oder in einem Abstand von höchstens der halben Lange der Einführeinrichtung (5) kreuzt.

24. Insertionskopf nach einem der voranstehenden Ansprüche, wobei in der Aufnahme (10, 14), die die Einführeinrichtung (5, 15) in der Schutzposition aufnimmt, eine Sicherungskulisse (50) vorgesehen ist.

25. Insertionskopf nach Anspruch 24, wobei die Sicherungskulisse in eine der Einführeinrichtung (5, 15) zugeordnete der Schutzposition nahen Sperrposition verbringbar ist.

26. Insertionskopf nach Anspruch 25, wobei der Sperrposition eine Rastschulter (50a) zugeordnet ist.

27. Insertionskopf nach einem der Ansprüche 17 bis 26, wobei die bewegliche Griffkomponente (12) in die weitere Griffkomponente (10) soweit einschiebbar ist, dass eine Steuerrampe (42) an der beweglichen Griffkomponente (12) nach Beendigung des Vorgangs zur Überführung der Einführeinrichtung (5, 15) von der Schutzposition in die Einführposition gegen eine Auslenkrampe (54) an einem Verbindungselement (16) anstößt, das die Basis (1) reversibel mit dem Griff (10, 12) verbindet, aus einer Eingriffsstellung auslenkt, so dass der Griff und die Basis trennbar sind.

28. Insertionskopf nach einem der Ansprüche 17 bis 27, wobei die Aufnahme (14) und die weitere Griffkomponente (10) einstückig sind, wobei die Aufnahme nicht Bestandteil der Basis (1) ist.

29. Insertionskopf nach einem der Ansprüche 17 bis 28, wobei der Einführeinrichtung eine Haltestruktur (17) zugeordnet ist, die mit der Einführeinrichtung mitbewegt wird, wobei die Haltesstruktur (17) eine Rastschulter (60) aufweist, die beim Herausziehen der beweglichen Griffkomponente (12) aus der weiteren Griffkomponente (10) in einen verrastenden Eingriff mit einem Eingriffelement (48) an der beweglichen Griffkomponente tritt, um ein neuerliches Herausschwenken der Einstecheinrichtung (15) zu verhindern.

30. Insertionskopf nach einem der Ansprüche 17 bis 29, wobei der Einführeinrichtung eine Haltestruktur (17) zugeordnet ist, die mit der Einführeinrichtung mitbewegt wird, wobei die Haltestruktur (17) eine Sicherungsstruktur (46) umfasst, die zu einer parallel zu der Bewegungsrichtung der beweglichen Griffkomponente (12) erstreckten Wand der beweglichen Griffkomponente (12) gegenüberliegt, wobei ein Eingriffselement (48) an der Innenseite dieser Wand mit der Sicherungsstruktur (46) in der Schutzposition der Einführeinrichtung (5, 15) im reversiblen Eingriff steht.

31. Insertionskopf nach einem der Ansprüche 17 bis 30, mit den folgenden Merkmalen:
- die Einführeinrichtung (5, 15) ist über eine Drehachse (6) an der Basis (1) gehalten;
- ein Kulissenstein (44) ist vorgesehen, der in Bezug auf die Drehachse (6) azentrisch angeordnet ist;
- eine Kulissenführung (42) ist vorgesehen, die im Wirkzusammenhang mit dem Kulissenstein (44) steht;
- wobei eine Bewegung der beweglichen Griffkomponente (12) relativ zu der Basis über eine erste Strecke (a) eines ersten Abschnitts (42a) der Kulissenführung, den 1. Kulissenstein (44), geführt durch die Kulissenführung (42), aus einer ersten Stellung, die der Schutzposition der Einführeinrichtung (5, 15) entspricht, in eine zweite Stellung, die der Einführposition der Einführeinrichtung (5) entspricht, überführt.

32. Insertionskopf nach Anspruch 31, wobei eine Verbindungseinrichtung (16b) vorgesehen ist, die die Basis (1) reversibel mit dem Griff (10, 12) verbindet, wobei bevorzugt zum Losen des Griffs von der Basis insbesondere die bewegliche Griffkomponente über eine zweite Strecke (b) senkrecht zur Erstreckungsrichtung der in der Einführposition befindlichen Einführeinrichtung (5, 15) bewegbar ist, wobei bevorzugt die Kulissenführung (42) einen zu der zweiten Strecke (b) korrespondierenden zweiten Abschnitt (42b) aufweist.

33. Insertionskopf nach einem der Ansprüche 1 bis 32, wobei der Einführeinrichtung (5, 15) eine Sicherungsstruktur zugeordnet ist, die die Einführeinrichtung (5, 15) in der Schutzposition reversibel sichert.

34. Insertionskopf nach Anspruch 33, wobei der Einführeinrichtung ein Kanülengehäuse (17b) zugeordnet ist, die mit der Einführeinrichtung mitbewegt wird, wobei das Kanülengehäuse (17b) eine Sicherungsstruktur (46) umfasst, die zu einer parallel zu der Bewegungsrichtung der zweiten Griffkomponente (12) erstreckten Wand der beweglichen Griffkomponente (12) gegenüberliegt, wobei ein Eingriffselement (48) an der Innenseite dieser Wand mit der Sicherungsstruktur (46) in der Schutzposition der Einführeinrichtung (5, 15) im reversiblen Eingriff steht.

35. Insertionskopf nach einem der voranstehenden Ansprüche, wobei in der weiteren Griffkomponente (10) und/oder der Aufnahme (14) und/oder in der zweiten Griffkomponente (12), die die Einführeinrichtung (15) in der Schutzposition aufnimmt, eine Sicherungskulisse (50) vorgesehen ist, in die die Einführeinrichtung (5, 15) in der Schutzposition einrastbar ist.

36. Insertionskopf nach einem der voranstehenden Ansprüche, wobei die bewegliche Griffkomponente (12) in die weitere Griffkomponente (10) soweit einschiebbar ist, dass eine Steuerrampe (52) an der beweglichen Griffkomponente (12) nach Beendigung des Vorgangs zur Überführung der Einführeinrichtung (5, 15) von der Schutzposition in die Einführposition gegen eine Auslenkrampe (54) an einem Verbindungselement (16) anstößt, das die Basis (1) reversibel mit dem Griff (10, 12) verbindet, aus einer Eingriffsstellung auslenkt, so dass der Griff und die Basis trennbar sind.

37. Insertionskopf nach einem der voranstehenden Ansprüche, wobei der Einführeinrichtung eine Haltestruktur (17) zugeordnet ist, die mit der Einführeinrichtung mitbewegt wird, wobei die Haltesstruktur (17) eine Rastschulter (60) aufweist, die bei einer Relativbewegung der beweglichen Griffkomponente (12) gegenüber der weiteren Griffkomponente (10) in einen verrastenden Eingriff mit einem Eingriffelement (48) tritt, um ein neuerliches Herausschwenken der Einstecheinrichtung (15) zu verhindern.

## Claims

1. An insertion head for medical or pharmaceutical applications, said insertion head comprising:
a) a base (1, 2) having a lower side (U) that can be positioned on tissue,
b) an insertion device (5) that can be inserted into the tissue, wherein the insertion device (5) is permanently and movably mounted on the base (1, 2) and wherein the base (1, 2) and a joint element (6) together form a joint and the insertion device (5) projects from the joint element (6), preferably in that a longitudinal direction of the insertion device (5) intersects or crosses a movement axis of the joint,
c) a receptacle (14) which is open to the lower side (U) of the base (1, 2) and is configured to be detached from said base (1, 2), and which receives at least one free end of the insertion device (5) in a protective position,
d) a handle (11, 12, 14) which is detachably connected to the base (1, 2) and the insertion device (5) mounted on the base (1, 2) and forms the detachable receptacle (14);
wherein
e) the insertion device (5) is pivotable relative to the base (1, 2) from its protective position in which a free end of the insertion device (5) stands back behind the lower side (U) of the base (1, 2) into an insertion position in which the free end of the insertion device (5) projects beyond the lower side (U) of the base (1, 2).

2. The insertion head according to claim 1, **characterized in that** the base (1, 2) creates an additional receptacle (1) which is open to the lower side (U) of the base (1, 2) and which receives a portion of the insertion device (5) in the insertion position thereof, and that the insertion device (5) projects through a recess (4) of the additional receptacle (1) into the detachable receptacle (14).

3. The insertion head according to the preceding claim, **characterized in that** the detachable receptacle (14) lengthens the additional receptacle (1) in the longitudinal direction of the insertion device (5), preferably receives the additional receptacle (1).

4. The insertion head according to one of the two preceding claims, **characterized in that** the additional receptacle (1) comprises an outer surface which slopes outwardly in a round pre-curved manner from its upper side towards the lower side (U) of the base (1, 2).

5. The insertion head according to any of the preceding claims, **characterized in that** the handle (11, 12, 14) comprises a handle part (11, 12) which projects from an upper side of the base (1, 2) and the detachable receptacle (14) projects laterally from the handle part (11, 12).

6. The insertion head according to any of the preceding claims, **characterized in that** the handle (11, 12, 14) comprises a handle component (12) which is movable relative to the base (1, 2) or is formed as such and the movable handle component (12) is coupled with the insertion device (5) by means of a transmission (8, 18) in such a manner that a movement of the movable handle component (12) taking place relative to the base (1, 2) effects the movement of the insertion device (5).

7. The insertion head according to the preceding claim, **characterized in that** the transmission (8, 18) couples the movable handle component (12) to the insertion device (5) by means of a toothed engagement, preferably a gearwheel (8) and a gear rod (18).

8. The insertion head according to any of the two preceding claims, **characterized in that** the movable handle component (12) is connected with a gear rod (18) which is preferably formed on the movable handle component (12) and the insertion device (5) to a gearwheel (8) which is in toothed engagement with the gear rod (18).

9. The insertion head according to any of the three preceding claims, **characterized in that** the insertion device (5) is connected in a pivotable and torsion-proof manner about an axis of rotation to a gearwheel (8) which is pivotable about the axis of rotation and in toothed engagement.

10. The insertion head according to any of the preceding claims, **characterized in that** the handle (11, 12, 14) has a handle component (12) being relatively movable to the base (1, 2) and a connection existing between the base (1, 2) and the handle (11, 12) is released during a movement of the movable handle component (12) .

11. The insertion head according to the preceding claim, **characterized in that** a first connecting element (9) is formed on the base (1, 2) and a second connecting element (19) is formed on the handle (11, 12, 14), that the connecting elements (9, 19) are in an engagement with one another which creates the connection and that at least one of the connecting elements (9, 19) can preferably be moved out of the engagement against an elasticity force.

12. The insertion head according to any of the two preceding claims, **characterized in that** a first connecting element (9) and a second connecting element (19) are in an engagement with one another which creates the connection and the movable handle component (12) contacts one of the connecting elements (9, 19) during its movement and preferably moves out of the engagement against an elasticity force.

13. The insertion head according to any of claims 7 to 13, **characterized in that** the handle (11, 12, 14) comprises a further handle component (11, 14) and the movable handle component (12) and the additional handle component (11, 14) can be gripped between two fingers of a hand, wherein with one finger in each case pressing against one of the handle components (11, 12, 14), the movable handle component (12) is movable against the additional handle component (11, 14) .

14. The insertion head according to any of claims 6 to 13, **characterized in that** the movable handle component (12) is displaceable relative to the base (1, 2).

15. The insertion head according to any of claims 6 to 14, **characterized in that** the movable handle component (12) is linearly guided.

16. The insertion head according to any of claims 6 to 15, **characterized in that** the movable handle component (12) is movable at least essentially parallel to the lower side (U) of the base (1, 2).

17. The insertion head according to any of claims 6 to 16, **characterized in that** the handle (11, 12, 14) comprises a further handle component (11, 14) which is unmovable relative to the base (1, 2) in the connected state and forms the detachable receptacle (14).

18. The insertion head according to any of the preceding claims, **characterized in that** the handle (11, 12, 14) projects from the upper side of the base (1, 2).

19. The insertion head according to any of the preceding claims, **characterized in that** the insertion device (5) is flexible and stabilized by an injection device (15) which is movable along with the insertion device (5) from the protective position into the insertion position.

20. The insertion head according to the preceding claim, **characterized in that** the injection device (15) is connected to the detachable receptacle (14) during movement of the insertion device (5) into the insertion position.

21. The insertion head according to any of the two preceding claims, **characterized in that** the injection device (15) with the detachable receptacle (14) can be detached from the base (1, 2) and the insertion device (5) .

22. The insertion head according to any of the preceding claims, **characterized in that** the insertion device (5) is elongated in a longitudinal direction and the longitudinal direction and the lower side (U) of the base (1, 2) enclose an acute angle of less than 50° when the free end of the insertion device (5) protrudes beyond the lower side (U) as it moves into the insertion position.

23. The insertion head according to any of the preceding claims, **characterized in that** the insertion device (5) is mounted on the base (1, 2) in a pivotable manner about an axis of rotation and the insertion device (5) has a longitudinal axis which intersects the axis of rotation or crosses it at a distance of no more than half the length of the insertion device (5).

24. The insertion head according to any of the preceding claims, **characterized in that** a securing link (50) is provided in the receptacle (10, 14) which receives the insertion device (5, 15) in the protective position.

25. The insertion head according to claim 24, **characterized in that** the securing link can be moved into a locking position which is associated with the insertion device (5, 15) and is close to the protective position.

26. The insertion head according to claim 25, **characterized in that** the locking position is assigned a latching collar (50a).

27. The insertion head according to any of claims 17 to 26, **characterized in that** the movable handle component (12) can be shifted so far into the additional handle component (10) that once the process for transferring the insertion device (5, 15) from the protective position into the insertion position has been completed, a control ramp (42) on the movable handle component (12) abuts against a deflecting ramp (54) on a connecting element (16) which reversibly connects the base (1) to the handle (10, 12), and deflects out of an engagement position, such that the handle and the base can be separated.

28. The insertion head according to any of claims 17 to 27, **characterized in that** the receptacle (14) and the additional handle component (10) are integral, wherein the receptacle is not a constituent part of the base (1) .

29. The insertion head according to any of claims 17 to 28, wherein the insertion device is associated with a holding structure (17) which is moved with the insertion device, wherein the holding structure (17) comprises a latching collar (60) which, when the movable handle component (12) is drawn out of the additional handle component (10), enters into a latching engagement with an engaging element (48) on the movable handle component, in order to prevent the injection device (15) from pivoting out again.

30. The insertion head according to any of claims 17 to 29, **characterized in that** the insertion device is associated with a holding structure (17) which is moved with the insertion device, wherein the holding structure (17) comprises a securing structure (46) which lies opposite a wall of the movable handle component (12) which extends parallel to the movement direction of the movable handle component (12), wherein an engaging element (48) on the inner side of this wall is in reversible engagement with the securing structure (46) when the insertion device (5, 15) is in its protective position.

31. The insertion head according to any of claims 17 to 30 having the following features:
- the insertion device (5, 15) is held on the base (1) via a rotational axis (6);
- a link block (44) is provided which is arranged non-centrically in relation to the axis of rotation (6),
- a link guide (42) is provided which cooperates with the link block (44);
- wherein a movement of the movable handle component (12) relative to the base over a first path (a) of a first portion (42a) of the link guide transfers the first link block (44), guided by the link guide (42), from a first position which corresponds to the protective position of the insertion device (5, 15) into a second position which corresponds to the insertion position of the insertion device (5).

32. The insertion head according to claim 31, **characterized in that** a connecting device (16b) is provided which reversibly connects the base (1) to the handle (10, 12), wherein preferably in order to detach the handle from the base, the movable handle component is in particular movable over a second path (b) perpendicular to the extending direction of the insertion device (5, 15) situated in the insertion position, wherein the link guide (42) preferably comprises a second portion (42b) corresponding to the second path (b).

33. The insertion head according to any of claims 1 to 32, **characterized in that** the insertion device (5, 15) is associated with a securing structure which reversibly secures the insertion device (5, 15) in the protective position.

34. The insertion head according to claim 33, **characterized in that** the insertion device is associated with a cannula casing (17b) which is moved with the insertion device, wherein the cannula casing (17b) comprises a securing structure (46) which lies opposite a wall of the movable handle component (12) which extends parallel to the movement direction of the a second handle component (12), wherein an engaging element (48) on the inner side of this wall is in reversible engagement with the securing structure (46) when the insertion device (5, 15) is in the protective position.

35. The insertion head according to any of the preceding claims, **characterized in that** a securing link (50) is provided in the additional handle component (10) and/or the receptacle (14) and/or in the second handle component (12) which receives the insertion device (15) in the protective position, with which securing link the insertion device (5, 15) can engage in the protective position.

36. The insertion head according to any of the preceding claims, **characterized in that** the movable handle component (12) can be shifted so far into the additional handle component (10) that once the process for transferring the insertion device (5, 15) from the protective position into the insertion position has been completed, a control ramp (52) on the movable handle component (12) abuts against a deflecting ramp (54) on a connecting element (16) which reversibly connects the base (1) to the handle (10, 12), and deflects out of an engagement position, such that the handle and the base can be separated.

37. The insertion head according to any of the preceding claims, **characterized in that** the insertion device is associated with a holding structure (17) which is moved with the insertion device, wherein the holding structure (17) comprises a latching collar (60) which, when there is a relative movement of the movable handle component (12) in respect of the additional handle component (10), enters into a latching engagement with an engaging element (48), in order to prevent the injection device (15) from pivoting out again.

## Revendications

1. Tête d'insertion pour des applications médicales ou pharmaceutiques, la tête d'insertion comprenant :
a) une base (1, 2) avec une sous-face (U) pouvant être placée sur du tissu organique,
b) un dispositif d'introduction (5) pouvant être introduit dans le tissu, dans laquelle la base (1, 2) supporte le dispositif d'introduction (5) de manière permanente et mobile et dans laquelle la base (1, 2) et un élément d'articulation (6) forment ensemble une articulation et le dispositif d'introduction (5) dépasse de l'élément d'articulation (6), de préférence en ce qu'un sens longitudinal du dispositif d'introduction (5) coupe ou croise un axe de déplacement de l'articulation,
c) une réception (14) séparable ouverte en direction de la sous-face (U) de la base (1, 2) et séparable de la base (1, 2) qui reçoit au moins une extrémité libre du dispositif d'introduction (5) dans une position de protection,
d) une poignée (11, 12, 14) qui est reliée de manière séparable à la base (1, 2) et au dispositif d'introduction (5) supporté par la base (1, 2) et forme la réception (14) séparable ;
dans laquelle
e) le dispositif d'introduction (5), par rapport à la base (1, 2) à partir de sa position de protection dans laquelle une extrémité libre du dispositif d'introduction (5) est en retrait derrière la sous-face (U) de la base (1, 2), peut pivoter dans une position d'introduction dans laquelle l'extrémité libre du dispositif d'introduction (5) fait saillie sur la sous-face (U) de la base (1, 2).

2. Tête d'insertion selon la revendication 1, **caractérisée en ce que** la base (1, 2) forme une réception supplémentaire (1) ouverte en direction de la sous-face (U) de la base (1, 2) qui reçoit un tronçon du dispositif d'introduction (5) dans sa position d'introduction et que le dispositif d'introduction (5) fait saillie à travers un évidement (4) de la réception supplémentaire (1) dans la réception séparable (14).

3. Tête d'insertion selon la revendication précédente, **caractérisée en ce que** la réception séparable (14) prolonge la réception supplémentaire (1) dans le sens longitudinal du dispositif d'introduction (5), reçoit de préférence la réception supplémentaire (1).

4. Tête d'insertion selon une des deux revendications précédentes, **caractérisée en ce que** la réception supplémentaire (1) présente une surface extérieure qui descend en étant bombée vers l'avant en arrondi vers l'extérieur, de sa face supérieure en direction de la sous-face (U) de la base (1, 2).

5. Tête d'insertion selon l'une des revendications précédentes, **caractérisée en ce que** la poignée (11, 12, 14) présente une partie de poignée (11, 12) faisant saillie d'une face supérieure de la base (1, 2) et la réception (14) séparable fait saillie latéralement de la partie de poignée (11, 12).

6. Tête d'insertion selon l'une des revendications précédentes, **caractérisée en ce que** la poignée (11, 12, 14) comprend une composante de poignée mobile (12) par rapport à la base (1, 2) ou est formée en tant que telle et la composante de poignée mobile (12) est ainsi couplée au dispositif d'introduction (5) au moyen d'un engrenage (8, 18) qu'un déplacement de la composante de poignée mobile (12) qui se produit par rapport à la base (1, 2) a pour effet le déplacement du dispositif d'introduction (5).

7. Tête d'insertion selon la revendication précédente, **caractérisée en ce que** l'engrenage (8, 18) couple la composante de poignée mobile (12) au dispositif d'introduction (5) au moyen d'un engrènement des dents, de préférence d'un pignon (8) et d'une crémaillère (18).

8. Tête d'insertion selon l'une des deux revendications précédentes, **caractérisée en ce que** la composante de poignée mobile (12) est reliée à une crémaillère (18) qui est formée de préférence sur la composante de poignée mobile (12), et le dispositif d'introduction (5) est relié à un pignon (8) se trouvant dans l'engrènement des dents avec la crémaillère (18).

9. Tête d'insertion selon l'une des trois revendications précédentes, **caractérisée en ce que** le dispositif d'introduction (5) est relié pivotant sur un axe de rotation et rigide en rotation à un pignon (8) se trouvant dans l'engrènement des dents, pouvant tourner sur l'axe de rotation.

10. Tête d'insertion selon l'une des revendications précédentes, **caractérisée en ce que** la poignée (11, 12, 14) présente une composante de poignée mobile (12) par rapport à la base (1, 2) et une liaison existant entre la base (1, 2) et la poignée (11, 12) se sépare lors d'un déplacement de la composante de poignée mobile (12).

11. Tête d'insertion selon la revendication précédente, **caractérisée en ce qu'**un premier élément de liaison (9) est formé sur la base (1, 2) et un second élément de liaison (19) est formé sur la poignée (11, 12, 14), que les éléments de liaison (9, 19) sont en engrènement entre eux formant la liaison et qu'au moins un des éléments de liaison (9, 19) peut se déplacer hors de l'engrènement de préférence contre une force d'élasticité.

12. Tête d'insertion selon l'une des deux revendications précédentes, **caractérisée en ce qu'**un premier élément de liaison (9) et un second élément de liaison (19) sont en engrènement entre eux formant la liaison et la composante de poignée mobile (12) contacte un des éléments de liaison (9, 19) lors de son déplacement et se déplace hors de l'engrènement de préférence contre une force d'élasticité.

13. Tête d'insertion selon l'une des revendications 7 à 13, **caractérisée en ce que** la poignée (11, 12, 14) présente une composante de poignée supplémentaire (11, 14) et la composante de poignée mobile (12) et la composante de poignée supplémentaire (11, 14) sont préhensibles entre deux doigts d'une main, dans laquelle avec un des doigts appuyant contre une des composantes de poignée (11, 12, 14), la composante de poignée mobile (12) peut être déplacée contre la composante de poignée supplémentaire (11, 14).

14. Tête d'insertion selon l'une des revendications 6 à 13, **caractérisée en ce que** la composante de poignée mobile (12) peut être décalée par rapport à la base (1, 2).

15. Tête d'insertion selon l'une des revendications 6 à 14, **caractérisée en ce que** la composante de poignée mobile (12) est dirigée de façon linéaire.

16. Tête d'insertion selon l'une des revendications 6 à 15, **caractérisée en ce que** la composante de poignée mobile (12) est mobile au moins essentiellement parallèlement à la sous-face (U) de la base (1, 2).

17. Tête d'insertion selon l'une des revendications 6 à 16, **caractérisée en ce que** la poignée (11, 12, 14) comprend une composante de poignée supplémentaire (11, 14) qui est immobile par rapport à la base (1, 2) dans l'état lié et forme la réception (14) séparable.

18. Tête d'insertion selon l'une des revendications précédentes, **caractérisée en ce que** la poignée (11, 12, 14) fait saillie d'une face supérieure de la base (1, 2).

19. Tête d'insertion selon l'une des revendications précédentes, **caractérisée en ce que** le dispositif d'introduction (5) est flexible et stabilisé par un dispositif de piquage (15) qui est mobile conjointement avec le dispositif d'introduction (5) de la position de protection à la position d'introduction.

20. Tête d'insertion selon la revendication précédente, **caractérisée en ce que** le dispositif de piquage (15) se lie avec la réception (14) séparable lors du déplacement du dispositif d'introduction (5) dans la position d'introduction.

21. Tête d'insertion selon l'une des deux revendications précédentes, **caractérisée en ce que** le dispositif de piquage (15) avec la réception (14) séparable est séparable d'avec la base (1, 2) et le dispositif d'introduction (5).

22. Tête d'insertion selon l'une des revendications précédentes, **caractérisée en ce que** le dispositif d'introduction (5) est étiré en longueur dans un sens longitudinal et le sens longitudinal inclut avec la sous-face (U) de la base (1, 2) un angle aigu inférieur à 50° lorsque l'extrémité libre du dispositif d'introduction (5) avance au-dessus de la sous-face (U) lors du déplacement dans la position d'introduction.

23. Tête d'insertion selon l'une des revendications précédentes, **caractérisée en ce que** la base (1, 2) supporte le dispositif d'introduction (5) pivotant sur un axe de rotation et le dispositif d'introduction (5) présente un axe longitudinal qui coupe l'axe de rotation ou croise l'axe de rotation dans une distance d'au plus la demi-longueur du dispositif d'introduction (5).

24. Tête d'insertion selon l'une des revendications précédentes, dans laquelle une coulisse de fixation (50) est prévue dans la réception (10, 14) qui reçoit le dispositif d'introduction (5, 15) dans la position de protection.

25. Tête d'insertion selon la revendication 24, dans laquelle la coulisse de fixation peut être amenée dans une position de blocage proche de la position de protection attribuée au dispositif d'introduction (5, 15).

26. Tête d'insertion selon la revendication 25, dans laquelle un épaulement d'encliquetage (50a) est attribué à la position de blocage.

27. Tête d'insertion selon l'une des revendications 17 à 26, dans laquelle la composante de poignée mobile (12) peut être poussée si loin dans la composante de poignée supplémentaire (10) qu'une rampe de commande (42) sur la composante de poignée mobile (12), une fois le passage du dispositif d'introduction (5, 15) de la position de protection à la position d'introduction terminé, vient heurter une rampe de déviation (54) sur un élément de liaison (16) qui relie la base (1) de manière réversible à la poignée (10, 12), dévie hors d'une position d'engrènement de sorte que la poignée et la base sont séparables.

28. Tête d'insertion selon l'une des revendications 17 à 27, dans laquelle la réception (14) et la composante de poignée supplémentaire (10) sont d'un seul bloc, dans laquelle la réception ne fait pas partie de la base (1).

29. Tête d'insertion selon l'une des revendications 17 à 28, dans laquelle une structure de retenue (17) est attribuée au dispositif d'introduction qui est déplacée conjointement avec le dispositif d'introduction, dans laquelle la structure de retenue (17) présente un épaulement d'encliquetage (60) qui, lorsque la composante de poignée mobile (12) est sortie hors de la composante de poignée supplémentaire (10), avance en un engrènement d'encliquetage avec un élément d'engrènement (48) contre la composante de poignée mobile pour empêcher un nouveau pivotement sortant du dispositif de piquage (15).

30. Tête d'insertion selon l'une des revendications 17 à 29, dans laquelle une structure de retenue (17) est attribuée au dispositif d'introduction qui est déplacée conjointement avec le dispositif d'introduction, dans laquelle la structure de retenue (17) comprend une structure de fixation (46) qui est opposée à une paroi de la composante de poignée mobile (12) parallèle au sens de déplacement de la composante de poignée mobile (12), dans laquelle un élément d'engrènement (48) est en engrènement réversible avec la structure de fixation (46) dans la position de protection du dispositif d'introduction (5, 15) sur la face intérieure de cette paroi.

31. Tête d'insertion selon l'une des revendications 17 à 30, avec les attributs suivants :
- le dispositif d'introduction (5, 15) est maintenu contre la base (1) par le biais d'un axe tournant (6) ;
- un coulisseau (44) est prévu qui est disposé de manière acentrique par rapport à l'axe tournant (6) ;
- un guide à coulisse (42) est prévu qui est en relation fonctionnelle avec le coulisseau (44) ;
- dans laquelle un déplacement de la composante de poignée mobile (12) par rapport à la base sur une première distance (a) d'un premier tronçon (42a) du guide à coulisse, fait passer le 1^{er} coulisseau (44), dirigé à travers le guide à coulisse (42), d'une première position qui correspond à la position de protection du dispositif d'introduction (5, 15), à une deuxième position qui correspond à la position d'introduction du dispositif d'introduction (5).

32. Tête d'insertion selon la revendication 31, dans laquelle un dispositif de liaison (16b) est prévu qui relie la base (1) de manière réversible à la poignée (10, 12), dans laquelle de préférence pour séparer la poignée de la base, en particulier la composante de poignée mobile peut être déplacée sur une seconde distance (b) verticale au sens d'étirement du dispositif d'introduction (5, 15) se trouvant dans la direction d'introduction, dans laquelle de préférence, le guide à coulisse (42) présente un second tronçon (42b) correspondant à la seconde distance (b).

33. Tête d'insertion selon l'une des revendications 1 à 32, dans laquelle une structure de fixation est attribuée au dispositif d'introduction (5, 15) qui fixe de manière réversible le dispositif d'introduction (5, 15) dans la position de protection.

34. Tête d'insertion selon la revendication 33, dans laquelle un logement de canule (17b) est attribué au dispositif d'introduction, qui est déplacé conjointement avec le dispositif d'introduction, dans laquelle le logement de canule (17b) comprend une structure de fixation (46) qui est opposée à une paroi de la composante de poignée mobile (12) parallèle au sens de déplacement de la composante de poignée mobile (12), dans laquelle un élément d'engrènement (48) est en engrènement réversible avec la structure de fixation (46) dans la position de protection du dispositif d'introduction (5, 15) sur la face intérieure de cette paroi.

35. Tête d'insertion selon l'une des revendications précédentes, dans laquelle une coulisse de fixation (50) est prévue dans la composante de poignée supplémentaire (10) et/ou la réception (14) et/ou dans la seconde composante de poignée (12) qui reçoit le dispositif d'introduction (15) dans la position de protection, dans laquelle le dispositif d'introduction (5, 15) peut être encliqueté dans la position de protection.

36. Tête d'insertion selon l'une des revendications précédentes, dans laquelle la composante de poignée mobile (12) peut être poussée si loin dans la composante de poignée supplémentaire (10) qu'une rampe de commande (52) sur la composante de poignée mobile (12), une fois le passage du dispositif d'introduction (5, 15) de la position de protection à la position d'introduction terminé, vient heurter une rampe de déviation (54) sur un élément de liaison (16) qui relie la base (1) de manière réversible à la poignée (10, 12), dévie hors d'une position d'engrènement de sorte que la poignée et la base sont séparables.

37. Tête d'insertion selon l'une des revendications précédentes, dans laquelle une structure de retenue (17) est attribuée au dispositif d'introduction qui est déplacée conjointement avec le dispositif d'introduction, dans laquelle la structure de retenue (17) présente un épaulement d'encliquetage (60) qui, lors d'un déplacement relatif de la composante de poignée mobile (12) vis-à-vis de la composante de poignée supplémentaire (10) avance en un engrènement d'encliquetage avec un élément d'engrènement (48) pour empêcher un nouveau pivotement sortant du dispositif de piquage (15).
